# EUROPEAN PATENT APPLICATION

(11) **EP 4 674 839 A1**
(43) Date of publication of application: **07.01.2026**
(21) Application number: 24764198.8
(22) Date of filing: 28.02.2024
(51) Int. Cl.: C07D 209/36, A61K 31/404, A61K 31/496, A61K 31/5377, A61K 31/541, A61K 31/454, A61K 31/41, A61P 3/00, A61P 19/08, A61P 25/28

(54) **NOVEL INDOLINE DERIVATIVE AND USE THEREOF**

(30) Priority: 28.02.2023 KR 20230027241; 27.02.2024 KR 20240027917
(71) Applicant: CK Regeon Inc., Seodaemoon-gu Seoul 03722 (KR)
(72) Inventor: CHOI, Kang-Yell, Seoul 03174 (KR); YANG, Jee Sun, Goyang-si Gyeonggi-do 10494 (KR); KANG, Min-Jeong, Incheon 21408 (KR); CHO, Han-Jun, Seoul 03725 (KR); JEONG, Sun Yong, Seoul 03782 (KR); HAN, Gyoonhee, Hwaseong-si Gyeonggi-do 18376 (KR); KIM, Yeji, Seoul 03727 (KR)
(74) Representative: V.O.
(86) International application number: PCT/KR2024/002578
(87) International publication number: WO 2024/181799

(57) **Abstract**

The present disclosure relates to an indoline derivative that inhibits CXXC5-Dvl interaction, a preparation method thereof and a use thereof. The present disclosure has resolved the low solubility of a conventional indoline derivative and efficiently activates the Wnt/β-catenin signaling pathway through excellent activity by which CXXC5-Dvl interaction is inhibited, and thus can be effectively used for the effects of treating, alleviating or preventing diseases associated with CXXC5-Dvl interaction activity.

## Description

### [Technical Field]

The present disclosure relates to an indoline derivative that inhibits CXXC5-Dvl interaction, a method for preparing the same, and a use thereof.

### [Background Art]

CXXC5 (CXXC-type zinc finger protein 5) is a recently identified protein that inhibits the Wnt/β-catenin signaling pathway. CXXC5 has a domain (DBM, Dvl binding motif) that binds to the dishevelled (Dvl) protein at the C-terminal, through which it binds to Dvl and inhibits Wnt/β-catenin signaling. It was determined that overexpressed CXXC5 in disease situations inhibits bone growth and osteoblast differentiation by suppressing the Wnt/β-catenin signaling pathway, and also affects skin wound healing and collagen formation, and plays an important role in various diseases including neurodegenerative diseases and hair loss.

Through this process, the CXXC5-Dvl interaction can be an important target for the development of new drugs for diseases such as metabolic diseases, osteoporosis, bone growth, skin wound, neurodegenerative diseases, hair loss, etc. Although it is expected that the inhibition of the CXXC5-Dvl interaction would improve several pathophysiological phenotypes associated with Wnt/β-catenin signaling through activation of Wnt/β-catenin, to date, no compound has been developed that exhibits a robust mechanism of action that disrupts the CXXC5-Dvl interaction, has high utility as a therapeutic agent, and is clinically effective.

Against this background, the inventors of the present disclosure have searched for a compound useful as a drug, having the effect of inhibiting the CXXC5-Dvl interaction and having good physicochemical properties such as solubility, and have completed the present disclosure by confirming that an indoline derivative of the present disclosure can be used usefully.

### [Disclosure]

### [Technical Problem]

The present disclosure has been made to solve the above-described problems and is directed to providing an indoline derivative represented by Chemical Formula 1, and a pharmaceutical composition, a food composition or a cosmetic composition for preventing, treating or alleviating a disease associated with CXXC5-Dvl interaction without side effects on the human body, which contains the indoline derivative as an active ingredient.

In addition, the present disclosure is directed to providing a method for preparing the indoline derivative and a useful intermediate compound for preparing the same.

### [Technical Solution]

The present disclosure provides an indoline derivative represented by Chemical Formula 1, a hydrate thereof, a solvate thereof, or a pharmaceutically acceptable salt thereof.

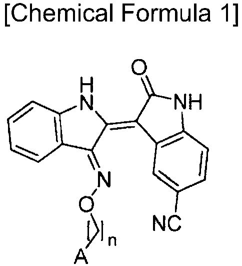

In Chemical Formula 1,
A is a 5- to 7-membered aromatic or non-aromatic heterocyclic group containing 1 to 3 heteroatoms selected from O, N, S, SO and SO₂;
the aromatic or non-aromatic heterocyclic group may be substituted or unsubstituted with one or more substituents selected from the group consisting of hydrogen, a halogen group, a hydroxyl group (-OH), a carboxyl group, and a C₁₋₆ alkyl group; and
n is an integer from 1 to 4.

In Chemical Formula 1, A may be a 5- to 6-membered non-aromatic heterocyclic group containing 1 to 2 heteroatoms selected from O, N, S, SO and SO₂, and the non-aromatic heterocyclic group may be substituted or unsubstituted with one or more substituents selected from hydrogen, a carboxyl group, and a C₁₋₆ alkyl group.

In Chemical Formula 1, A may be selected from substituted or unsubstituted pyrrolidine, substituted or unsubstituted piperidine, substituted or unsubstituted piperazine, substituted or unsubstituted morpholine, substituted or unsubstituted thiomorpholine, and substituted or unsubstituted proline.

The indoline derivative of Chemical Formula 1 may be any one selected from the group consisting of 5-cyanoindirubin-3'-piperazinylethyloxime; 5-cyanoindirubin-3'-morpholinylethyloxime; 5-cyanoindirubin-3'-thiomorpholinylethyloxime; 5-cyanoindirubin-3'-(4-methylpiperazinyl)ethyloxime; 5-cyanoindirubin-3'-piperidinylethyloxime; 5-cyanoindirubin-3'-pyrrolidinylethyloxime; 5-cyanoindirubin-3'-piperidinylpropyloxime; 5-cyanoindirubin-3'-pyrrolidinylpropyloxime; 5-cyanoindirubin-3'-piperazinylpropyloxime; 5-cyanoindirubin-3'-morpholinylpropyloxime; 5-cyanoindirubin-3'-(4-methylpiperazinyl)propyloxime; 5-cyanoindirubin-3'-thiomorpholinylpropyloxime; 5-cyanoindirubin-3'-(1,1-dioxothiomorpholinyl)ethyloxime; 5-cyanoindirubin-3'-(1-oxothiomorpholinyl)ethyloxime hydrochloride; and 5-cyanoindirubin-3'-(2-prolinyl)ethyloxime.

The indoline derivative represented by Chemical Formula 1 may be any one selected from the group consisting of 5-cyanoindirubin-3'-piperazinylethyloxime; 5-cyanoindirubin-3'-morpholinylethyloxime; 5-cyanoindirubin-3'-thiomorpholinylethyloxime; and 5-cyanoindirubin-3'-(4-methylpiperazinyl)ethyloxime.

The present disclosure also provides a method for preparing the compound represented by Chemical Formula 1, which includes:
1) a step of replacing the hydrogen atom of the hydroxyl group of an indirubin oxime derivative represented by Chemical Formula 2 with a C₁₋₆ alkyl group substituted with a C₁₋₆ haloalkyl group, bromine (Br), a tosyl group (OTs), or a cyano group; and
2) a step of adding a 5- to 7-membered aromatic or non-aromatic heterocyclic compound containing 1 to 3 heteroatoms selected from O, N, S, SO and SO₂ to the resultant product of the step 1) to obtain a compound represented by Chemical Formula 1.

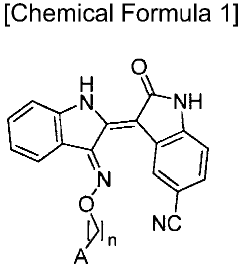

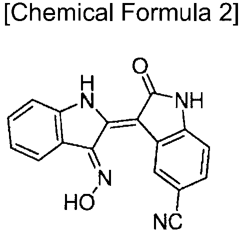

In Chemical Formula 1,
A is a 5- to 7-membered aromatic or non-aromatic heterocyclic group containing 1 to 3 heteroatoms selected from O, N, S, SO and SO₂; and
the aromatic or non-aromatic heterocyclic group may be substituted or unsubstituted with one or more substituents selected from the group consisting of hydrogen, a halogen group, a hydroxyl group (-OH), a carboxyl group, and a C₁₋₆ alkyl group; and n is an integer from 1 to 4.

The indirubin oxime derivative represented by Chemical Formula 2 may be synthesized through the following steps:
A) a step of reacting 3-indoxyl acetate and an isatin compound represented by Chemical Formula 3 in a solvent to obtain an intermediate compound represented by Chemical Formula 4; and
B) a step of mixing the intermediate compound represented by Chemical Formula 4 with hydroxylamine to obtain an indirubin oxime compound represented by Chemical Formula 2.

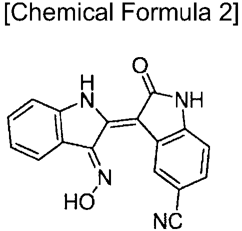

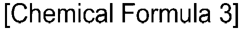

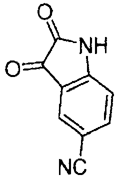

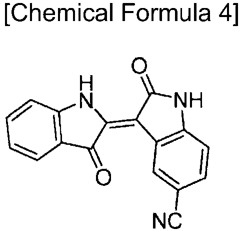

The present disclosure also provides a pharmaceutical composition for preventing or treating a disease associated with CXXC5-Dvl interaction, which contains the indoline derivative or a pharmaceutically acceptable salt thereof as an active ingredient.

The disease associated with CXXC5-Dvl interaction may be at least one selected from the group consisting of metabolic diseases, bone diseases, neurodegenerative diseases, hair loss, and wound.

The metabolic disease may be at least one selected from the group consisting of diabetes, diabetic complications, obesity, hyperlipidemia, fatty liver, arteriosclerosis, nephropathy, hepatopathy, hypertension, stroke, heart failure, renal failure, dyslipidemia, and insulin resistance syndrome, the bone disease may be at least one selected from the group consisting of osteoporosis, osteoarthritis, osteogenesis imperfecta, bone defects, osteoclastosis, osteomalacia, osteoarthritis, rheumatoid arthritis, and fracture, and the neurodegenerative disease may be at least one selected from the group consisting of Parkinson's disease, stroke, spinal cord injury, ischemic brain disease, epilepsy, Alzheimer's disease, dementia, depression, bipolar disorder, and schizophrenia.

The diabetic complication is at least one selected from the group consisting of diabetic nephropathy, diabetic hepatopathy, diabetic wound, diabetic foot ulcers, hypertension, arteriosclerosis, stroke, and heart failure.

The fatty liver is also called steatotic liver, and the fatty liver disease may be at least one selected from alcoholic fatty liver, alcoholic steatohepatitis, nonalcoholic fatty liver, nonalcoholic steatohepatitis, and hepatocirrhosis and cirrhosis caused by steatohepatitis. As re-defined terms, the fatty liver may also be one or more selected from the group consisting of metabolic dysfunction-associated steatotic liver disease (MASLD), metabolic dysfunction-associated steatohepatitis (MASH), MAFLD-associated hepatocirrhosis, and cirrhosis.

The composition may be administered orally.

The present disclosure also provides a food composition for preventing or alleviating a disease associated with CXXC5-Dvl interaction, which contains the indoline derivative as an active ingredient.

The present disclosure also provides a cosmetic composition for preventing or alleviating a disease associated with CXXC5-Dvl interaction, which contains the indoline derivative as an active ingredient.

### [Advantageous Effects]

A novel indoline derivative according to the present disclosure improves the low solubility of existing indoline derivatives and efficiently activates the Wnt/β-catenin signaling pathway through excellent activity of inhibiting CXXC5-Dvl interaction. Therefore, it can be easily utilized for the treatment, alleviation or prevention of diseases related to the CXXC5-Dvl interaction activity.

The novel indoline derivative according to the present disclosure can restore the Wnt/β-catenin signaling system by directly and specifically inhibit the binding of CXXC5 and Dvl protein in vivo and, therefore, can very effectively treat related diseases such as metabolic diseases, bone diseases, neurodegenerative diseases, hair loss, and wound.

Since the novel indoline derivative according to the present disclosure is a safe substance that is non-toxic to cells, it can be usefully used in a pharmaceutical composition or a food composition.

### [Brief Description of Drawings]

FIG. 1 shows a result of analyzing the concentration-dependent CXXC5-Dvl binding inhibition activity of indoline derivatives of Examples 1 to 15, 6-chloro-5-methylindirubin-3'-(4-methylpiperazinyl)ethyloxime, an intermediate compound (5-cyanoindirubin), and an indirubin compound.
FIG. 2 shows a result of comparing the Oil Red O staining images and lipid accumulation amounts showing the effects of indoline derivatives of Examples 1 to 15, an intermediate compound (5-cyanoindirubin), A3051, A3334, I30, BIO, and an indirubin compound on adipocyte differentiation using 3T3-L1 pre-adipocytes.
FIG. 3 is a schematic diagram that outlines the experimental design of MASH (metabolic dysfunction-associated steatohepatitis) in Test Example 3.
FIG. 4 shows an insulin tolerance test (ITT) result for a MASH group (vehicle) and test groups 1 to 5.
FIG. 5 shows a result of histological analysis of morphologically observing liver tissues extracted from a normal group (NCD), a MASH group (vehicle), and test groups 1 to 5, and staining the same with hematoxylin and eosin (H&E).
FIG. 6 shows a result of extracting liver tissues from a normal group (NCD), a MASH group (vehicle), and test groups 1 to 5, and evaluating the MAFLD (metabolic dysfunction-associated fatty liver disease) activity score to confirm histological changes.
FIG. 7 shows a result of Oil Red O staining (ORO) and immunohistochemical staining (PPARγ) showing the expression of adipocyte differentiation markers in liver tissues extracted from a normal group (NCD), a MASH group (vehicle), and test groups 1 to 5.
FIG. 8 shows a result of immunohistochemical staining confirming the increased expression of inflammatory markers (CD11b and F4/80) in liver tissues extracted from a normal group (NCD), a MASH group (vehicle), and test groups 1 to 5.
FIG. 9 shows a result of immunohistochemical staining confirming the increased expression of fibrosis markers (α-SMA, collagen I, and desmin) in liver tissues extracted from a normal group (NCD), a MASH group (vehicle), and test groups 1 to 5.
FIG. 10 shows a result of analyzing the degree of liver fibrosis for a normal group (NCD), a MASH group (vehicle), and test groups 1 to 5.
FIG. 11 shows a result of immunohistochemical staining analysis of the expression level of β-catenin and CXXC5 in a normal group (NCD), a MASH group (vehicle), and test groups 1 to 5.
FIGS. 12 and 13 show the H&E staining images of adipose tissues isolated from a normal group (NCD), a MASH group (vehicle), and test groups 1 to 5. FIG. 12 shows eWAT (epididymal white adipose tissue), and FIG. 13 shows iWAT (inguinal white adipose tissue).

### [Best Mode]

Hereinafter, various aspects and exemplary embodiments of the present disclosure will be described in more detail.

In this specification, when a part is said to 'include' a certain component, this means that it may include other components rather than excluding other components, unless the context specifically states otherwise.

The Wnt/β-catenin signaling pathway is known to be involved in metabolic diseases, bone diseases, neurodegenerative diseases, hair loss, and wound. CXXC-type zinc finger protein 5 (CXXC5) is a negative regulator of Wnt/β-catenin signaling that functions through interaction with the dishevelled (Dvl) PDZ domain in the cytoplasm. Therefore, the inhibition of the CXXC5-Dvl interaction can restore downregulated Wnt/β-catenin signaling caused by overexpresssion of CXXC5, leading to treatment, prevention or alleviation of the pathophysiological phenotypes of various diseases associated with the Wnt/β-catenin signaling pathway. In other words, to discover inhibitors of the CXXC5-Dvl interaction is a key to the treatment of metabolic diseases, bone diseases, neurodegenerative diseases, alopecia, and wound.

Accordingly, the inventors of the present disclosure screened substances that inhibit the CXXC5-Dvl interaction, and analyzed whether they treat or alleviate the symptoms induced by diseases while specifically targeting the binding of CXXC5 and Dvl in vivo while. As a result, they have completed the present disclosure by discovering small-molecule compounds based on indoline derivatives that actually have excellent therapeutic uses.

An aspect of the present disclosure relates to an indoline derivative represented by Chemical Formula 1, a hydrate thereof, a solvate thereof, or a pharmaceutically acceptable salt thereof.

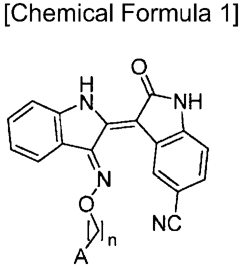

In Chemical Formula 1,
A is a 5- to 7-membered aromatic or non-aromatic heterocyclic group containing 1 to 3 heteroatoms selected from O, N, S, SO and SO₂; the aromatic or non-aromatic heterocyclic group may be substituted or unsubstituted with one or more substituents selected from the group consisting of hydrogen, a halogen group, a hydroxyl group (-OH), a carboxyl group and a C₁₋₆ alkyl group; and n is an integer from 1 to 4.

According to an exemplary embodiment of the present disclosure, in Chemical Formula 1, A is a 5- to 6-membered non-aromatic heterocyclic group containing 1 to 2 heteroatoms selected from O, N, S, SO and SO₂, and the non-aromatic heterocyclic group may be substituted or unsubstituted with one or more substituents selected from hydrogen, a carboxyl group and a C₁₋₆ alkyl group.

In Chemical Formula 1, A may be any one selected from Structural Formula 1-1 or Structural Formula 1-2.

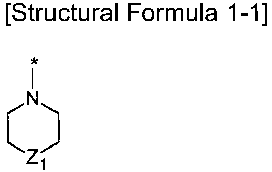

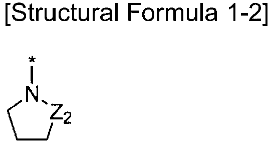

In Structural Formulas 1-1 and 1-2,
each of Z₁ and Z₂ is independently any one selected from C, CR', S, SR', SR'R", O, N and NR', and each of R' and R" may independently be selected from the group consisting of hydrogen, =O, -OH, -COOH and a C₁₋₆ alkyl group.

Specifically, in Chemical Formula 1, A may be selected from substituted or unsubstituted pyrrolidine, substituted or unsubstituted piperidine, substituted or unsubstituted piperazine, substituted or unsubstituted morpholine, substituted or unsubstituted thiomorpholine, and substituted or unsubstituted proline, and the substituent may be any one selected from a halogen and a C₁₋₆ alkyl group.

More specifically, in Chemical Formula 1, A may be any one selected from piperidine, pyrrolidine, morpholine, thiomorpholine, 1,1-dioxothiomorpholine, 1-oxothiomorpholine, piperazine, N-methylpiperazine, and proline, although not being limited thereto.

The indoline derivative of Chemical Formula 1 may be any one selected from the group consisting of 5-cyanoindirubin-3'-piperazinylethyloxime; 5-cyanoindirubin-3'-morpholinylethyloxime; 5-cyanoindirubin-3'-thiomorpholinylethyloxime; 5-cyanoindirubin-3'-(4-methylpiperazinyl)ethyloxime; 5-cyanoindirubin-3'-piperidinylethyloxime; 5-cyanoindirubin-3'-pyrrolidinylethyloxime; 5-cyanoindirubin-3'-piperidinylpropyloxime; 5-cyanoindirubin-3'-pyrrolidinylpropyloxime; 5-cyanoindirubin-3'-piperazinylpropyloxime; 5-cyanoindirubin-3'-morpholinylpropyloxime; 5-cyanoindirubin-3'-(4-methylpiperazinyl)propyloxime; 5-cyanoindirubin-3'-thiomorpholinylpropyloxime; 5-cyanoindirubin-3'-(1,1-dioxothiomorpholinyl)ethyloxime; 5-cyanoindirubin-3'-(1-oxothiomorpholinyl)ethyloxime; and 5-cyanoindirubin-3'-(2-prolinyl)ethyloxime, although not being limited thereto.

The indoline derivative represented by Chemical Formula 1 may specifically be any one selected from the group consisting of 5-cyanoindirubin-3'-piperazinylethyloxime; 5-cyanoindirubin-3'-morpholinylethyloxime; 5-cyanoindirubin-3'-thiomorpholinylethyloxime; and 5-cyanoindirubin-3'-(4-methylpiperazinyl)ethyloxime. Most specifically, it may be 5-cyanoindirubin-3'-piperazinylpropyloxime; or 5-cyanoindirubin-3'-morpholinylpropyloxime in terms of the effect of restoring liver health.

In the present disclosure, the "heterocyclic group" refers to a group that contains one or more non-carbon atoms or heteroatoms. Specifically, the heteroatom may be one to three heteroatoms selected from the group consisting of O, N, S, SO and SO₂. The number of carbon atoms is not particularly limited, but the heterocyclic group may be a 5- to 7-membered non-aromatic heterocyclic group. Examples of the heterocyclic group include a thiophene group, a furan group, a pyrrole group, an imidazole group, a thiazole group, an oxazole group, an oxadiazole group, a pyridyl group, a bipyridyl group, a pyrimidyl group, a triazine group, a triazole group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, an isoquinoline group, an indole group, a carbazole group, a benzoxazole group, a benzimidazole group, a benzothiazole group, a benzocarbazole group, a benzothiophene group, a dibenzothiophene group, a benzofuranyl group, a phenanthroline group, a thiazolyl group, an isoxazolyl group, an oxadiazolyl group, a thiadiazolyl group, a benzothiazolyl group, a phenothiazinyl group, a dibenzofuranyl group, a substituted or unsubstituted pyrrolidine group, a substituted or unsubstituted piperidine group, a substituted or unsubstituted piperazine group, a substituted or unsubstituted morpholine group, a substituted or unsubstituted thiomorpholine group, a substituted or unsubstituted proline group, etc., although not being limited thereto.

The term "substitution" means that a hydrogen atom bonded to a carbon atom is replaced with another substituent, and the position of the substitution is not limited as long as it is the position at which the hydrogen atom is replaced, i.e., the position at which the substitution can be made. When two or more substitutions are made, the two or more substituents may be the same or different from each other.

The term "substituted or unsubstituted" as used herein, unless defined otherwise, means substituted with one or two or more substituents selected from the group consisting of hydrogen; deuterium; a halogen group; a hydroxyl group; a C₁₋₆ alkyl group; and a C₁₋₆ alkoxy group, substituted with a substituent in which two or more of the above-mentioned substituents are linked, or having no substituent at all.

For example, the aromatic or non-aromatic heterocyclic group may be substituted or unsubstituted with one or more substituents selected from the group consisting of hydrogen, a halogen group, a hydroxyl group (-OH), a carboxyl group, and a C₁₋₆ alkyl group, as defined above.

In the present disclosure, the "halogen" refers to elements belonging to group 17 of the periodic table. Since there are 7 electrons in the outermost electron shell, they easily become anions by obtaining electrons from other elements. They are the most non-metallic in each period, and mainly exist in the form of compounds with other elements because they are highly reactive. They include fluorine, chlorine, bromine, iodine, etc.

In the present disclosure, the "hydroxyl group" refers to a functional group with the structural formula -OH.

In the present disclosure, "alkyl" generally means a straight-chain or branched-chain saturated hydrocarbon group having a specified number of carbon atoms (e.g., 1 to 12, specifically 1 to 6 carbon atoms). The alkyl group may be any one selected from the group consisting of methyl, ethyl, propyl, 1-methylethyl, butyl, 1-methylpropyl, 2-methylpropyl, 1,1-dimethylethyl, n-pentyl, 1-methylbutyl, 2-methylbutyl, 3-methylbutyl, 1,2-dimethylpropyl, 1,1-dimethylpropyl, 2,2-dimethylpropyl, 1-ethylpropyl, n-hexyl, 1-methylpentyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, 1-ethyl-2-methylpropyl, n-heptyl, 1-methylpexyl, 1-ethylpentyl, 2-ethylpentyl, 1-propylbutyl, octyl, decyl and dodecyl, although not being limited thereto.

The term "alkoxy" in the present disclosure means a functional group having - OR, wherein an alkyl group described above is attached to a parent compound via an oxygen atom. Examples of the C₁₋₆ alkoxy group include, but are not limited to, methoxy, ethoxy, propoxy, isopropoxy, butoxy, isobutoxy, pentoxy, and hexoxy. In the present disclosure, "haloalkoxy" refers to a group in which one hydrogen atom of an alkoxy group described above has been replaced with a halogen element.

According to an exemplary embodiment of the present disclosure, the indoline derivative represented by Chemical Formula 1 may be represented by any one selected from a group consisting of Chemical Formulas 1-1 to 1-15.

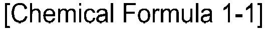

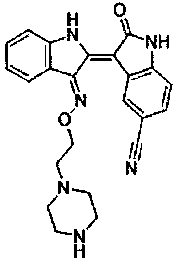

The compound name of the indoline derivative of Chemical Formula 1-1 is 5-cyanoindirubin-3'-piperazinylethyloxime (CK001), and its IUPAC name is (2Z,3E)-2'-oxo-3-((2-(piperazin-1-yl)ethoxy)imino)-[2,3'-biindolinylidene]-5'-carbonitrile.

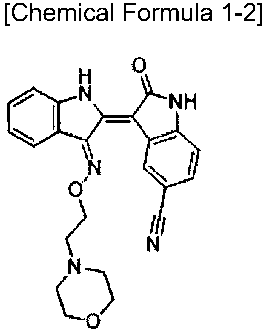

The compound name of the indoline derivative of Chemical Formula 1-2 is 5-cyanoindirubin-3'-morpholinylethyloxime (CK002), and its IUPAC name is (2Z,3E)-3-((2-morpholinoethoxy)imino)-2'-oxo-[2,3'-biindolinylidene]-5'-carbonitrile.

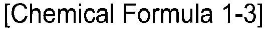

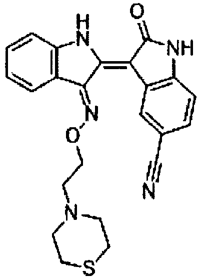

The compound name of the indoline derivative of Chemical Formula 1-3 is 5-cyanoindirubin-3'-thiomorpholinylethyloxim (CK003), and its IUPAC name is (2Z,3E)-2'-oxo-3-((2-thiomorpholinoethoxy)imino)-[2,3'-biindolinylidene]-5'-carbonitrile.

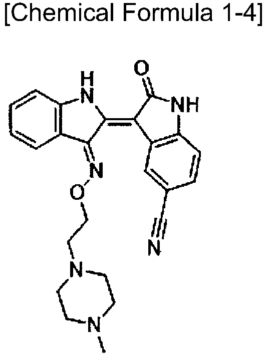

The compound name of the indoline derivative of Chemical Formula 1-4 is 5-cyanoindirubin-3'-(4-methylpiperazinyl)ethyloxime (CK004), and its IUPAC name is (2Z,3E)-3-((2-(4-methylpiperazin-1-yl)ethoxy)imino)-2'-oxo-[2,3'-biindolinylidene]-5'-carbonitrile.

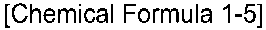

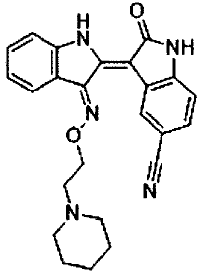

The chemical name of the indoline derivative of Chemical Formula 1-5 is 5-cyanoindirubin-3'-piperidinylethyloxime (CK005), and its IUPAC name is (2Z,3E)-2'-oxo-3-((2-(piperidin-1-yl)ethoxy)imino)-[2,3'-biindolinylidene]-5'-carbonitrile.

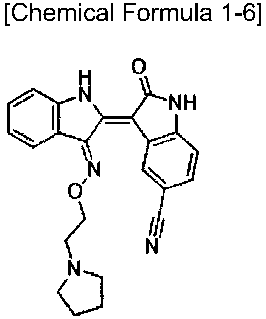

The compound name of the indoline derivative of Chemical Formula 1-6 is 5-cyanoindirubin-3'-pyrrolidinylethyloxime (CK006), and its IUPAC name is (2Z,3E)-2'-oxo-3-((2-(pyrrolidin-1-yl)ethoxy)imino)-[2,3'-biindolinylidene]-5'-carbonitrile.

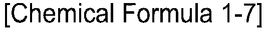

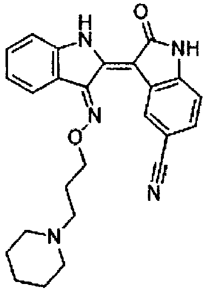

The compound name of the indoline derivative of Chemical Formula 1-7 is 5-cyanoindirubin-3'-piperidinylpropyloxime (CK007), and its IUPAC name is (2Z,3E)-2'-oxo-3-((3-(piperidin-1-yl)propoxy)imino)-[2,3'-biindolinylidene]-5'-carbonitrile.

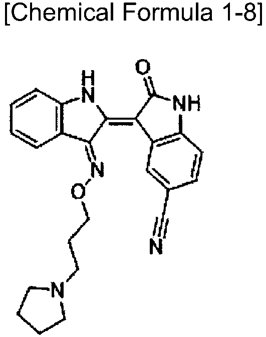

The compound name of the indoline derivative of Chemical Formula 1-8 is 5-cyanoindirubin-3'-pyrrolidinylpropyloxime (CK008), and its IUPAC name is (2Z,3E)-2'-oxo-3-((3-(pyrrolidin-1-yl)propoxy)imino)-[2,3'-biindolinylidene]-5'-carbonitrile.

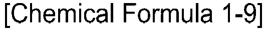

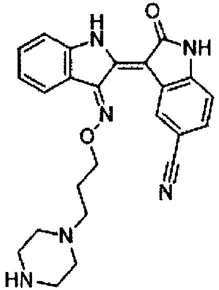

The compound name of the indoline derivative of Chemical Formula 1-9 is 5-cyanoindirubin-3'-piperazinylpropyloxime (CK009), and its IUPAC name is (2Z,3E)-2'-oxo-3-((3-(piperazin-1-yl)propoxy)imino)-[2,3'-biindolinylidene]-5'-carbonitrile.

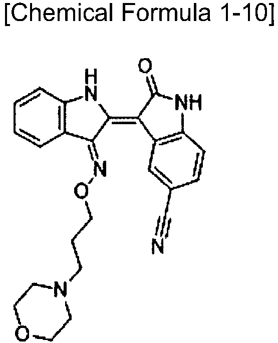

The compound name of the indoline derivative of Chemical Formula 1-10 is 5-cyanoindirubin-3'-morpholinylpropyloxime (CK010), and its IUPAC name is (2Z,3E)-3-((3-morpholinopropoxy)imino)-2'-oxo-[2,3'-biindolinylidene]-5'-carbonitrile.

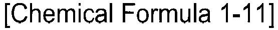

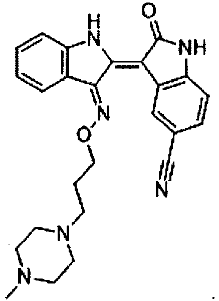

The compound name of the indoline derivative of Chemical Formula 1-11 is 5-cyanoindirubin-3'-(4-methylpiperazinyl)propyloxime (CK011), and its IUPAC name is (2Z,3E)-3-((3-(4-methylpiperazin-1-yl)propoxy)imino)-2'-oxo-[2,3'-biindolinylidene]-5'-carbonitrile.

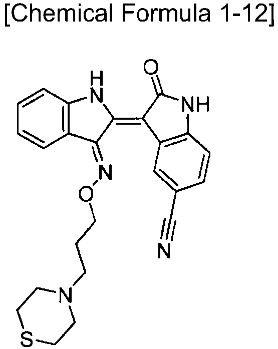

The compound name of the indoline derivative of Chemical Formula 1-12 is 5-cyanoindirubin-3'-thiomorpholinylpropyloxime (CK012), and its IUPAC name is (2Z,3E)-2'-oxo-3-((3-thiomorpholinopropoxy)imino)-[2,3'-biindolinylidene]-5'-carbonitrile.

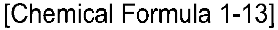

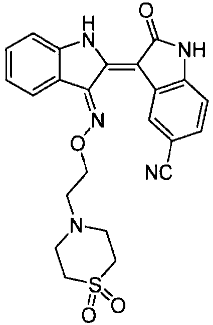

The compound name of the indoline derivative of Chemical Formula 1-13 is 5-cyanoindirubin-3'-(1,1-dioxothiomorpholinyl)ethyloxime (CK013), and its IUPAC name is (2Z,3E)-3-((2-(1,1-dioxidothiomorpholino)ethoxy)imino)-2'-oxo-[2,3'-biindolinidene]-5'-carbonitrile.

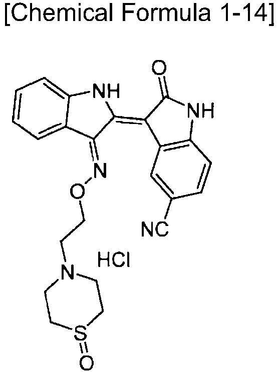

The compound name of the indoline derivative of Chemical Formula 1-14 is 5-cyanoindirubin-3'-(1-oxothiomorpholinyl)ethyloxime hydrochloride (CK014), and its IUPAC name is (2Z,3E)-3-((2-(1-oxidothiomorpholino)ethoxy)imino)-2'-oxo-[2,3'-biindolinidene]-5'-carbonitrile hydrochloride.

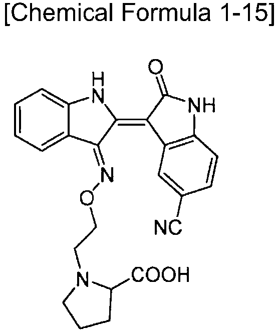

The compound name of the indoline derivative of Chemical Formula 1-15 is 5-cyanoindirubinoxime-3'-(2-prolinyl)ethyloxime (CK015), and its IUPAC name is (2-((((2Z,3E)-5'-cyano-2'-oxo-[2,3'-biindolinidene]-3-ylidene)amino)oxy)ethyl)proline.

In comparative examples, indirubin and 6-chloro-5-methylindirubin-3'-(4-methylpiperazinyl)ethyloxime were used. They are represented by Chemical Formulas 1-16 and 1-17, respectively.

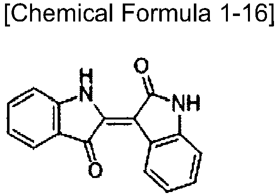

The compound name of the compound represented by Chemical Formula 1-16 is indirubin, and its IUPAC name is (Z)-[2,3'-biindolinylidene]-2',3-dione.

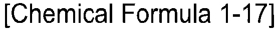

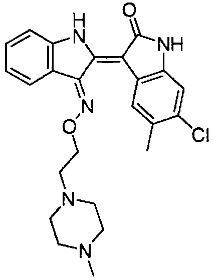

The compound name of the indoline derivative of Chemical Formula 1-17 is 6-chloro-5-methylindirubin-3'-(4-methylpiperazinyl)ethyloxime (CK090), and its IUPAC name is (2Z,3E)-6'-chloro-5'-methyl-3-((2-(4-methylpiperazin-1-yl)ethoxy)imino)-[2,3'-biindolinylidene]-2'-one.

Meanwhile, the indoline derivative represented by Chemical Formula 1 of the present disclosure can be used in the form of a pharmaceutically acceptable salt. As the salt, an acid addition salt formed from a pharmaceutically acceptable free acid is useful.

As the "salt" used in the present disclosure, an acid addition salt formed from a pharmaceutically acceptable free acid is useful. The acid addition salt is obtained from an inorganic acid such as hydrochloric acid, nitric acid, phosphoric acid, sulfuric acid, hydrobromic acid, hydroiodic acid, nitrous acid or phosphorous acid, or from a non-toxic organic acid such as an aliphatic mono- or dicarboxylate, a phenyl-substituted alkanoate, a hydroxyalkanoate, an alkanedioate, an aromatic acid, and an aliphatic or aromatic sulfonic acid. The pharmaceutically non-toxic salt includes: sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate or mandelate.

The acid addition salt according to the present disclosure can be prepared by a conventional method, for example, by dissolving a compound in an excess amount of an aqueous acid solution and precipitating a salt using a water-miscible organic solvent, e.g., methanol, ethanol, acetone or acetonitrile. It can also be prepared by evaporating the solvent or excess acid from the mixture and then drying or suction-filtering the precipitated salt.

Additionally, a pharmaceutically acceptable metal salt may be prepared using a base. An alkali metal or alkaline earth metal salt is obtained, for example, by dissolving a compound in an excess amount of an alkali metal hydroxide or alkaline earth metal hydroxide solution, filtering off an undissolved compound salt, and evaporating and drying the filtrate. For pharmaceutical purposes, it is preferred that a sodium, potassium or calcium salt is prepared as the metal salt. A silver salt is obtained by reacting an alkali metal or alkaline earth metal salt with a suitable salt (e.g., silver nitrate).

In addition, the indoline derivative of the present disclosure includes not only the pharmaceutically acceptable salt, but also all salts, isomers, hydrates and solvates that can be prepared by conventional methods.

In the present disclosure, the 'hydrate' refers to the compound of the present disclosure or a salt thereof, which contains a stoichiometric or non-stoichiometric amount of water bound by non-covalent intermolecular force. The hydrate of the indoline derivative represented by Chemical Formula 1 of the present disclosure may contain a stoichiometric or non-stoichiometric amount of water bound by non-covalent intermolecular forces. The hydrate may contain at least 1 equivalent, specifically 1 to 5 equivalents, of water. The hydrate may be prepared by crystallizing the indoline derivative represented by Chemical Formula 1 of the present disclosure, an isomer thereof, or a pharmaceutically acceptable salt thereof in water or a solvent containing water.

In the present disclosure, the "solvate" means a compound solvated in an organic or inorganic solvent, and means a higher-order compound formed between the molecule or ion of a solute and the molecule or ion of a solvent.

As can be confirmed in the examples described below, the indoline derivative represented by Chemical Formula 1 can be used as a CXXC5-Dvl interaction inhibitor, and as described above in the background art section, it is a widely known fact to those skilled in the art that CXXC-Dvl interaction inhibitors can be used for the treatment of metabolic diseases, bone diseases, neurodegenerative diseases, alopecia, and wound.

Some of the compounds according to the present disclosure can be prepared or synthesized through the following steps. Another aspect of the present disclosure relates to a method for preparing an indoline derivative represented by Chemical Formula 1, which includes:
1) a step of replacing the hydrogen atom of a hydroxyl group of the indirubin oxime derivative represented by Chemical Formula 2 with a C₁₋₆ alkyl group substituted with a C₁₋₆ haloalkyl group, a tosyl group (OTs) or a cyano group; and
2) a step of obtaining an indoline derivative represented by Chemical Formula 1 by adding a 5- to 7-membered aromatic or non-aromatic heterocyclic compound containing 1 to 3 heteroatoms selected from O, **N,** S, SO and SO₂ to the resultant product of the step 1).

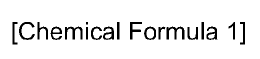

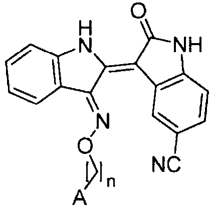

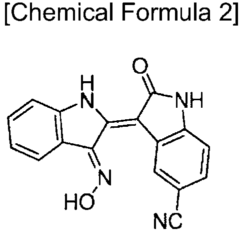

In Chemical Formula 1, A is a 5- to 7-membered aromatic or non-aromatic heterocyclic group containing 1 to 3 heteroatoms selected from O, N, S, SO and SO₂; the aromatic or non-aromatic heterocyclic group may be substituted or unsubstituted with one or more substituents selected from the group consisting of hydrogen, a halogen group, a hydroxyl group (-OH), a carboxyl group, and a C₁₋₆ alkyl group; and n is an integer from 1 to 4.

In the present disclosure, the "haloalkyl" refers to a group in which one hydrogen atom of an alkyl group is replaced with a halogen element. Depending on the number of carbons attached to the carbon to which the halogen is attached, it can also be named as primary, secondary, or tertiary haloalkyl. Specifically, 1 to 3 halogen atoms may be present. Specific examples thereof include difluoromethyl or trifluoromethyl groups. In the present disclosure, "halo" can be used interchangeably with "halogen".

In the step 1), the hydroxyl group hydrogen atom of the indirubin oxime derivative represented by Chemical Formula 2 can be replaced with a C₁₋₆ alkyl group substituted with a C₁₋₆ haloalkyl group, a tosyl group (OTs) or a cyano group.

The reaction temperature in the step 1) is not particularly limited, the reaction may be performed specifically at 20 to 100 °C, more specifically at 50 to 100 °C when the substitution is made with a haloalkyl group or a tosyl group (OTs), and at 20 to 100 °C when the substitution is made with a cyano group. After heating, the reaction mixture may be cooled to 10 to 30 °C.

In the step 2), 3 to 10 equivalents, specifically 5 equivalents, of a 5 to 7-membered aromatic or non-aromatic heterocyclic compound containing 1 to 3 heteroatoms selected from O, N, S, SO and SO₂ can be added based on 1 equivalent of the resultant product of the step 1).

In the step 2), the heterocyclic compound may be a compound containing one or more non-carbon atoms, or heteroatoms. Specifically, the compound may contain 1 to 3 heteroatoms selected from O, N, S, SO and SO₂. Although the number of carbon atoms is not particularly limited, the compound may be a compound having a 5- to 7-membered aromatic or non-aromatic heterocyclic ring. The heterocyclic compound may be thiophene, furan, pyrrole, imidazole, thiazole, oxazole, oxadiazole, pyridyl, bipyridyl, pyrimidyl, triazine, triazole, acridyl, pyridazine, pyrazinyl, quinolinyl, quinazoline, quinoxalinyl, phthalazinyl, pyridopyrimidinyl, pyridopyrazinyl, pyrazino pyrazinyl, isoquinoline, indole, carbazole, benzoxazole, benzimidazole, benzothiazole, benzocarbazole, benzothiophene, dibenzothiophene, benzofuranyl, phenanthroline, thiazolyl, isoxazolyl, oxadiazolyl, thiadiazolyl, benzothiazolyl, phenothiazinyl, dibenzofuranyl, substituted or unsubstituted pyrrolidine, substituted or unsubstituted piperidine, substituted or unsubstituted piperazine, substituted or unsubstituted morpholine, substituted or unsubstituted thiomorpholine, substituted or unsubstituted proline, etc., although not being limited thereto.

The substitution means that the hydrogen atom bonded to a carbon atom is replaced with another substituent, and the position of the substitution is not limited as long as it is the position at which the hydrogen atom is replaced, i.e., the position at which the substitution can be made. When two or more substitutions are made, the two or more substituents may be the same or different from each other. The expression 'substituted or unsubstituted' means substituted with one or two or more substituents selected from a group consisting of hydrogen; deuterium; a halogen group; a hydroxyl group; a C₁₋₆ alkyl group; and a C₁₋₆ alkoxy group, substituted with a substituent in which two or more of the above-mentioned substituents are linked, or having no substituent at all. The aromatic or non-aromatic heterocyclic group may be substituted or unsubstituted with one or more substituents selected from the group consisting of hydrogen, a halogen group, a hydroxyl group (-OH), a carboxyl group, and a C₁₋₆ alkyl group.

The reaction temperature in the step 2) is not particularly limited. Specifically, heating may be performed at 50 to 150 °C under reflux. After the heating, the reaction mixture may be cooled to 10 to 30 °C.

The indirubin oxime derivative represented by Chemical Formula 2 may be synthesized through the following steps:
A) a step of reacting 3-indoxyl acetate with an isatin compound represented by Chemical Formula 3 in a solvent to obtain an intermediate compound represented by Chemical Formula 4; and B) a step of mixing the intermediate compound represented by Chemical Formula 4 with hydroxylamine to obtain an indirubin oxime compound represented by Chemical Formula 2.

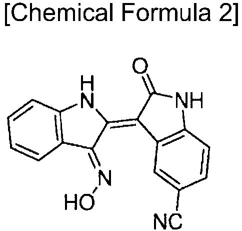

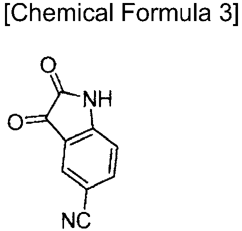

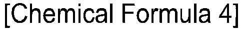

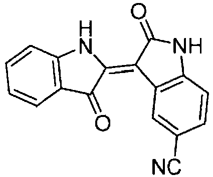

In the step A), the solvent may be any one selected from water, ethanol, propanol and methanol. In the step A), an acid may be further added. The acid is not particularly limited as long as it is an acidic substance. Specifically, hydrochloric acid or sulfuric acid may be used.

In the step A), the mixing molar ratio of the 3-indoxyl acetate and the isatin compound represented by Chemical Formula 3 is not particularly limited. They may be mixed at a ratio of 1:0.1 to 1:10, most specifically 1:1, in consideration of the reaction rate.

In the step A), the reaction temperature is not particularly limited. But, specifically, the reaction may be performed by heating at 60 to 100 °C under reflux. After the heating, the reaction mixture may be cooled to 10 to 30 °C.

The intermediate compound represented by Chemical Formula 4 prepared in the step A) is an intermediate compound of the compound represented by Chemical Formula 1. When an indoline derivative is prepared through this process, it is advantageous in that the synthesis procedure is simple.

In the step B), 2 to 10 equivalents, specifically 7 equivalents, of hydroxylamine or hydroxylamine hydrochloride may be mixed based on 1 equivalent of the intermediate compound represented by Chemical Formula 4.

The reaction temperature in the step B) is not particularly limited. But, specifically, the reaction may be performed by heating at 60 to 100 °C under reflux. After the heating, the reaction mixture may be cooled to 10 to 30 °C.

Another aspect of the present disclosure relates to an intermediate compound represented by Chemical Formula 4 for preparing the indoline derivative of Chemical Formula 1 or a part thereof.

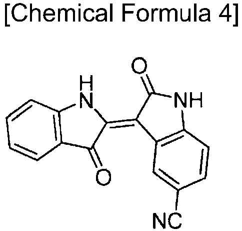

Another aspect of the present disclosure relates to a pharmaceutical composition for preventing or treating a disease associated with CXXC5-Dvl interaction, which contains the indoline derivative of Chemical Formula 1, a hydrate thereof, a solvate thereof, or a pharmaceutically acceptable salt thereof as an active ingredient.

The disease associated with CXXC5-Dvl interaction may be at least one selected from the group consisting of metabolic diseases, bone diseases, neurodegenerative diseases, hair loss, and wound, although not being limited thereto.

Since the compound according to the present disclosure has an excellent effect in inhibiting the binding of Dvl and CXXC5, it can be usefully used in preventing or treating diseases associated with the CXXC5-Dvl interaction, specifically metabolic diseases, bone diseases, neurodegenerative diseases, alopecia, or wound, although not being limited thereto.

In the present disclosure, the "metabolic disease" refers to a disease caused by excessive lipid accumulation in the body. As specific examples, it may be at least one selected from the group consisting of diabetes, diabetic complications, obesity, hyperlipidemia, fatty liver (or steatotic liver), arteriosclerosis, nephropathy, hepatopathy, hypertension, stroke, heart failure and renal failure, dyslipidemia, and insulin resistance syndrome.

In the present disclosure, the "diabetes" may be type 2 diabetes. The type 2 diabetes (T2DM) is a common chronic metabolic disease characterized by insulin resistance and relative deficiency of insulin secretion, which increases blood sugar levels. If this condition persists, it can cause serious damage to the heart, blood vessels, eyes, kidneys, and nerves. The symptoms of diabetes include increased thirst, weakness, fatigue, blurred vision, numbness or tingling in the hands or during eating, ulcers or wounds that are slow to heal, unplanned weight loss, frequent urination, dry mouth, etc.

In the present disclosure, the "diabetic complication" refers to side effects caused by diabetes. It may be one or more selected from a group consisting of diabetic nephropathy, diabetic hepatopathy, diabetic wound, diabetic foot ulcer, hypertension, arteriosclerosis, stroke, and heart failure, although not being particularly limited thereto.

In the present disclosure, the "obesity" refers to a condition or a disease in which excessive body fat has accumulated due to energy imbalance.

In the present disclosure, the "fatty liver" is also called steatotic liver. It refers to a disease in which normal fat metabolism is not achieved due to excessive fat intake and increased accumulation and synthesis of fat in the liver, decreased excretion of fat, etc., causing excessive presence of fat vacuoles in liver cells due to excessive accumulation of lipids, especially triglycerides.

The fatty liver disease or steatotic liver includes fatty liver, which is simple steatosis, steatohepatitis accompanied by inflammation, and hepatocirrhosis and cirrhosis caused thereby, and may be at least one selected from alcoholic fatty liver, alcoholic steatohepatitis, nonalcoholic fatty liver, nonalcoholic steatohepatitis, and hepatocirrhosis and cirrhosis caused by steatohepatitis.

With the recent redefinition of terms related to fatty liver disease, the term steatotic liver is already being used overseas. The fatty liver disease may be one or more selected from the group consisting of metabolic dysfunction-associated steatotic liver disease (MASLD), metabolic dysfunction-associated steatohepatitis (MASH), MAFLD-associated hepatocirrhosis, and cirrhosis. Hereinafter, the changed name will be used in the present disclosure.

The nonalcoholic steatohepatitis (NASH) is also called metabolic dysfunction-associated steatohepatitis (MASH). It is an advanced form of nonalcoholic fatty liver disease (NAFLD) (also called metabolic dysfunction-associated steatotic liver disease (MASLD)) caused by various metabolic disorders. MAFLD is the most common cause of chronic liver disease and is caused by the accumulation of fat in the liver. The underlying major causes of MAFLD include obesity, type 2 diabetes, dyslipidemia, and insulin resistance. If fat accumulates in the liver, inflammation and liver damage can occur, and MAFLD can progress to MASH, which makes the liver damage even worse. These changes in the liver can stimulate hepatic stellate cells, leading to fibrosis. As MASH progresses, it can cause portal hypertension, and 20% of patients progress to cirrhosis or liver cancer. MASH shows histological findings similar to those of alcoholic fatty liver disease even without a history of excessive drinking. Liver dysfunction is the most common cause of MASH, affecting up to 20% of the population, but there are no treatments for MASH approved by the U.S. Food and Drug Administration (FDA) or the European Commission (EC). Therefore, depending on the medical professional's judgment, drugs for diabetes, obesity, and hyperlipidemia are being used as off-label treatments. MASH is the most commonly diagnosed in patients between the ages of 40 and 60, but can occur at any age. Many MASH patients may show one or more of the following conditions: obesity, type 2 diabetes, glucose intolerance, dyslipidemia, and/or metabolic disease.

In the present disclosure, the "bone disease" results from the imbalance of osteoblasts in the bone, and examples thereof include, but are not limited to, at least one selected from the group consisting of osteoporosis, osteoarthritis, osteogenesis imperfecta, bone defects, osteoclastosis, osteomalacia, osteoarthritis, rheumatoid arthritis, and fracture.

In the present disclosure, the "neurodegenerative disease" refers to a disease that causes various symptoms as degenerative changes appear in nerve cells of the central nervous system. In most cases, the onset of the disease begins slowly and symptoms appear after a long period of normal function after birth. Additionally, once the disease develops, it progresses continuously over years or decades until death, and there is often a family history. Specific examples of the neurodegenerative disease of the present disclosure include, but are not limited to, at least one selected from the group consisting of Parkinson's disease, stroke, spinal cord injury, ischemic brain disease, epilepsy, Alzheimer's disease, dementia, depression, bipolar disorder, and schizophrenia. Among these, Alzheimer's disease is a disease that causes irreversible brain degeneration characterized by impairments in memory, cognition, personality, and other functions, ultimately leading to death due to complete brain failure. Genetic and environmental factors, including diet, activity, smoking, traumatic brain injury, diabetes, and other medical conditions, contribute to the risk of developing these diseases. While the commercially available medications can alleviate some of the symptoms associated with Alzheimer's disease, they do not provide solutions that slow the progression of the disease or correct the cause of the disease itself. Late-stage Parkinson's disease is another form of dementia and is characterized by cognitive deficits and behavioral changes that affect memory and learning ability, daily living, and quality of life. Parkinson's disease is characterized by the loss of dopamine-producing neurons in the brain. Some studies have reported that glucagon-like peptide-1 (GLP-1) is involved in the development of neurodegenerative diseases such as Alzheimer's disease and Parkinson's disease.

In the present disclosure, the "hair loss" refers to a state in which hair is absent in an area where hair should normally exist, regardless of the cause. It may be, for example, male pattern baldness, female pattern baldness, alopecia areata, telogen effluvium, stress-induced alopecia, or hair loss caused by chemotherapy agents. The composition of the present disclosure may be particularly effective in the treatment of male pattern baldness because it promotes proliferation of hair papilla cells and growth of hair length. In the present disclosure, the "hair loss" may be used interchangeably with "alopecia." Specifically, the hair loss may be at least one selected from the group consisting of androgenetic alopecia, alopecia areata, alopecia totalis, traction alopecia, cicatricial alopecia, telogen effluvium, age-related alopecia, and anagen effluvium.

In the present disclosure, the "wound" is also called a gash, and depending on the cause, it is divided into an incised wound, a cutting wound, a cleaver wound, a contused wound, a lacerated wound, a gunshot wound, a bite wound, etc., and depending on the shape, it is divided into a linear wound, a plate-shaped wound, a defect wound, etc. The symptoms of a wound include pain, bleeding, dysfunction, etc.

The wound may also be at least one selected from the group consisting of the burn of the epidermis, dermis or subcutaneous tissue of the skin, an ulcer, a trauma, a surgical operation, a plastic surgery, an implant, a childbirth, a chronic wound, a damage caused by dermatitis, a burn ulcer, a bedsore, and a chronic dermatitis.

The composition of the present disclosure can prevent or treat metabolic diseases, bone diseases, neurodegenerative diseases, alopecia and/or wound healing by activating the Wnt/β-catenin signaling pathway by inhibiting the binding of CXXC5 and Dvl. Therefore, it can be usefully used for the prevention or treatment of metabolic diseases, bone diseases, neurodegenerative diseases, hair loss and/or wound caused by abnormal interaction between CXXC5 and Dvl.

In addition, it was confirmed that the indoline derivative according to the present disclosure does not induce death of animals or cause inflammation or other pathological symptoms in animal experiments.

That is, the present disclosure may provide a pharmaceutical composition for preventing or treating metabolic diseases, bone diseases, neurodegenerative diseases, alopecia and wound, a use of the indoline derivative of Chemical Formula 1 for treating the diseases, and a method for treating the diseases, which includes administering a therapeutically effective amount of the indoline derivative represented by Chemical Formula 1 to a subject.

The indoline derivative represented by Chemical Formula 1 of the present disclosure inhibits the binding of CXXC5 and Dvl so that they cannot interact with each other. Therefore, by inhibiting the interaction between CXXC5 and Dvl, the activity level of the Wnt/β-catenin signaling pathway can be restored. In addition, since it can exhibit the effect of treating and alleviating various symptoms related to MASH (fibrosis, obesity, adipose tissue accumulation, etc.) in a mouse model wherein MASH is induced with a high-fat diet and CCl₄, the indoline derivative of the present disclosure can be usefully used as an active ingredient for treating, preventing or alleviating not only metabolic diseases but also bone diseases, neurodegenerative diseases, alopecia, and wound, wherein CXXC5 is known to be overexpressed, as a CXXC5-Dvl interaction inhibitor.

The term "prevention" as used in the present disclosure means any action of suppressing or delaying the onset of a target disease by administering the pharmaceutical composition according to the present disclosure.

The term "treatment" as used in the present disclosure means any action by which the symptoms of a target disease are alleviated or beneficially changed by administering the pharmaceutical composition according to the present disclosure.

The pharmaceutical composition of the present disclosure may contain a pharmaceutically acceptable carrier in addition to the active ingredient. The pharmaceutically acceptable carrier includes those commonly used in formulations, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, although not being limited thereto. In addition to the above-described ingredients, a lubricant, a wetting agent, a sweetener, a flavoring agent, an emulsifier, a suspending agent, a preservative, etc. may also be included.

The pharmaceutical composition of the present disclosure can be administered orally or parenterally (e.g., intravenously, subcutaneously, intraperitoneally, or topically) depending on the intended method, and the dosage can be appropriately selected by those skilled in the art although it varies depending on the patient's condition and body weight, the degree of a disease, drug type, the route and time of administration. Specifically, it may be administered orally.

The pharmaceutical composition of the present disclosure is administered in a pharmaceutically effective amount. In the present disclosure, the "pharmaceutically effective amount" means an amount sufficient to treat a disease at a reasonable benefit/risk ratio applicable to medical treatment, and the effective dosage level can be determined based on the type and severity of the patient's disease, the activity of a drug, the sensitivity to the drug, the time of administration, the route and excretion rate of administration, the duration of treatment, concurrently used drugs, and other factors well known in the medical field. The pharmaceutical composition according to the present disclosure may be administered as an individual therapeutic agent or in combination with another therapeutic agent, may be administered sequentially or simultaneously with the conventional therapeutic agent, and may be administered singly or in multiple doses. Taking all of the above factors into consideration, it is important to administer an amount that can achieve the maximum effect with the minimum amount with no side effect, which can be easily determined by those skilled in the art.

Specifically, the effective amount of the pharmaceutical composition of the present disclosure may vary depending on the patient's age, sex, condition and body weight, the absorption rate, inactivation rate and excretion rate of the active ingredient in the body, the type of a disease, and concomitantly administered drugs. In general, an amount of 0.0001 to 1000 mg, specifically 0.001 to 500 mg, per 1 kg of body weight may be administered daily or every other day, or divided into 1 to 3 times a day. However, since the dosage may be increased or decreased depending on the route of administration, the severity of a disease, sex, body weight, age, etc., the above dosage does not limit the scope of the present disclosure in any way.

In the present disclosure, the term "subject" means a subject requiring treatment of a disease, and more specifically, a mammal such as human, a non-human primate, mouse, dog, cat, horse, cow, etc.

Another aspect of the present disclosure relates to a use of the indoline derivative represented by Chemical Formula 1 for preparing a pharmaceutical preparation having an effect of preventing or treating a disease associated with CXXC5-Dvl interaction.

Another aspect of the present disclosure relates to a method for preparing a pharmaceutical preparation having an effect of preventing or treating a disease associated with CXXC5-Dvl interaction, characterized by using the indoline derivative represented by Chemical Formula 1.

Another aspect of the present disclosure relates to a food composition for preventing or alleviating a disease associated with CXXC5-Dvl interaction, which contains the indoline derivative represented by Chemical Formula 1, a hydrate thereof, a solvate thereof, or a pharmaceutically acceptable salt thereof as an active ingredient. The specific description of the food composition is the same as that of the pharmaceutical composition for preventing or treating a disease associated with CXXC5-Dvl interaction, which contains the indoline derivative represented by Chemical Formula 1, a hydrate thereof, a solvate thereof, or a pharmaceutically acceptable salt thereof as an active ingredient.

In the present disclosure, the "alleviation" means any action that at least decreases a parameter related to the condition being treated, e.g., the degree of symptoms. The functional food composition can be used simultaneously with or separately from a drug for treatment before or after the onset stage of a disease in order to prevent or alleviate a disease associated with CXXC5-Dvl interaction.

The food composition according to the present disclosure may be formulated in the same manner as the pharmaceutical composition, and may be used as a functional food or may be added to various foods. The food to which the composition of the present disclosure can be added includes, for example, beverages, alcoholic beverages, confectionery, diet bars, dairy products, meat, chocolate, pizza, ramen, other noodles, gum, ice cream, vitamin complexes, health supplements, etc.

The food composition of the present disclosure may contain not only the above-mentioned active ingredients, but also ingredients commonly added during preparation of foods, e.g., proteins, carbohydrates, fats, nutrients, seasonings, and flavoring agents. Examples of the carbohydrate include common sugars such as monosaccharides, e.g., glucose, fructose, etc.; disaccharides, e.g., maltose, sucrose, oligosaccharides, etc.; and polysaccharides, e.g., dextrin, cyclodextrin, etc., and sugar alcohols such as xylitol, sorbitol, erythritol, etc. As the flavoring agent, natural flavoring agents [thaumatin, stevia extracts (e.g., rebaudioside A, glycyrrhizin, etc.]) and synthetic flavoring agents (saccharin, aspartame, etc.) can be used. For example, when the food composition of the present disclosure is prepared into a drink or a beverage, citric acid, high-fructose corn syrup, sugar, glucose, acetic acid, malic acid, fruit juice, and various plant extracts may be additionally included in addition to the active ingredient of the present disclosure.

The present disclosure provides a health functional food for preventing or alleviating a disease associated with CXXC5-Dvl interaction, which contains the active ingredient described above. The health functional food refers to a food prepared by adding the indoline derivative represented by Chemical Formula 1, a hydrate thereof, a solvate thereof, or a pharmaceutically acceptable salt thereof, as an active ingredient, to a foodstuff such as a beverage, a tea, a spice, a gum, confectionery, etc., or by preparing it in the form of a capsule, a powder or a suspension, which brings about specific health effects when consumed, but, unlike general drugs, is advantageous in that it has no side effect that can occur during long-term use of drugs since it is made from a foodstuff. The health functional food of the present disclosure obtained in this manner is very useful because it can be consumed on a daily basis. The amount of the active ingredient added to the health functional food cannot be uniformly regulated as it varies depending on the type of the health functional food. It may be added within a range that does not harm the original taste of the food, which is usually in the range of 0.01 to 50 wt%, specifically 0.1 to 20 wt%, with respect to the target food. In addition, in the case of a health functional food in the form of a pill, a granule, a tablet or a capsule, it is usually added in a range of 0.1 to 100 wt%, specifically 0.5 to 80 wt%. In a specific exemplary embodiment, the health functional food of the present disclosure may be in the form of a pill, a tablet, a capsule, or a beverage.

Since the indoline derivative represented by Chemical Formula 1, a hydrate thereof, a solvate thereof, or a pharmaceutically acceptable salt thereof of the present disclosure has the effect of inhibiting the CXXC5-Dvl interaction, it can be used for the purpose of preventing, alleviating or treating related diseases. Therefore, another aspect of the present disclosure provides a cosmetic composition for preventing or alleviating a disease associated with CXXC5-Dvl interaction, which contains the indoline derivative represented by Chemical Formula 1, a hydrate thereof, a solvate thereof, or a pharmaceutically acceptable salt thereof as an active ingredient. Specifically, the disease associated with the CXXC5-Dvl interaction may be at least one selected from the group consisting of metabolic diseases, bone diseases, neurodegenerative diseases, hair loss, and wound, although not being particularly limited thereto.

If the composition according to the present disclosure is a cosmetic composition, it contains the indoline derivative represented by Chemical Formula 1 as an active ingredient, and can be prepared in the form of a basic cosmetic composition, a color cosmetic composition, a hair or scalp product composition, a soap, etc., together with a pH adjuster, a binder, a surfactant, a metal ion-sequestering agent, a preservative, a bactericidal preservative, an anti-discoloration agent, a moisturizer, an opacifier, an antioxidant, a cleanser, an astringent, a solvent, an emulsification stabilizer, an emulsifier, a UV blocker, a viscosity reducing agent, a viscosity increasing agent, an antistatic agent, a colorant, a fragrance, a film-forming agent, a skin-protecting agent, a skin-softening agent, a skin-conditioning agent, a hair-conditioning agent, a suspending agent, an absorbent, etc. For example, the cosmetic composition of the present disclosure may be a formulation in the form of a akin lotion, a skin softener, a skin toner, an astringent toner, an emollient toner, a nourishing toner, an astringent, a lotion, a milk lotion, a moisturizing lotion, a nourishing lotion, a skin lotion, a body cream, a massage cream, a nourishing cream, a moisturizing cream, a shower cream, a sunscreen lotion, a sunscreen cream, a suntan cream, a hand cream, a skin cream, a face or body cream, a skin-whitening cream, a hand lotion, a hair lotion, a cosmetic cream, an oil, an essence, a nourishing essence, a pack, a soap (bath soap, liquid soap, beauty soap, medicated soap (non-medical), or cream soap), a shampoo, a cleanser (cleansing foam, cleansing lotion, cleansing cream, cleansing water, or soap), a mask pack, a body oil, a body lotion, a body cleanser, a treatment, a beauty liquid, an emulsion, a foundation, a lipstick, a makeup base, a pressed powder, a loose powder, a concealer, an eye shadow, a rinse, a hair conditioner, a hair gel, a hair vinegar, a teeth-whitening gel, a toothpaste, a scalp cleanser, a hair rinse, etc., although not being limited thereto.

The composition of the present disclosure may further contain a solvent commonly used in the preparation of cosmetic compositions, or an appropriate carrier, excipient or diluent.

The excipient may include, for example, a pH adjuster, a sweetener, a binder, a surfactant, a metal ion-sequestering agent, a preservative, a sterilizing preservative, an anti-discoloration agent, a moisturizer, an opacifier, an antioxidant, a detergent, an astringent, a solvent, an emulsification stabilizer, an emulsifier, a UV blocker, a viscosity-reducing agent, a viscosity-increasing agent, an antistatic agent, a colorant, a flavoring agent, a film-forming agent, a skin-protecting agent, a skin softener, a skin-conditioning agent, a hair-conditioning agent, a suspending agent, an absorbent, etc., although not being limited thereto. For example, when the cosmetic composition of the present disclosure is prepared into a cleanser or a soap, the cleanser or soap can be prepared easily by adding the indoline derivative represented by Chemical Formula 1 of the present disclosure to a conventional cleanser or soap base. The cosmetic composition may be prepared by adding the indoline derivative represented by Chemical Formula 1 of the present disclosure to a conventional cosmetic composition base. Additionally, synthetic or natural materials such as a flavoring, a chelating agent, a coloring agent, an antioxidant, a preservative, a protein, a mineral, a vitamin, etc. may be further added for the purpose of improving physical properties.

The type of the solvent that can be further added to the cosmetic composition of the present disclosure includes, for example, water, saline, DMSO, or a combination thereof, and the carrier, excipient or diluent includes purified water, an oil, a wax, a fatty acid, a fatty acid alcohol, a fatty acid ester, a surfactant, a wetting agent, a thickener, an antioxidant, a viscosity stabilizer, a chelating agent, a buffer, a lower alcohol, etc., although not being limited thereto. Additionally, a whitening agent, a moisturizer, a vitamin, a sunscreen, a perfume, a dye, an antibiotic, an antibacterial agent, or an antifungal agent may be further included as needed.

As the oil, hydrogenated vegetable oil, castor oil, cottonseed oil, olive oil, palm oil, jojoba oil, or avocado oil can be used. And, as the wax, beeswax, spermaceti, carnauba, candelilla, montan, ceresin, liquid paraffin, or lanolin can be used.

As the fatty acid, stearic acid, linoleic acid, linolenic acid, or oleic acid can be used. And, as the fatty acid alcohol, cetyl alcohol, octyl dodecanol, oleyl alcohol, panthenol, lanolin alcohol, stearyl alcohol, or hexadecanol can be used. And, as the fatty acid ester, isopropyl myristate, isopropyl palmitate, or butyl stearate can be used. As the surfactant, a cationic surfactant, an anionic surfactant or a nonionic surfactant known in the art can be used. Specifically, surfactants derived from natural products are preferred if possible.

In addition, it may contain absorbents, thickeners, antioxidants, etc., which are widely known in the cosmetics field, and the types and amounts of these are as known in the art.

The content of the active ingredient contained in the cosmetic composition of the present disclosure may be 0.00001 to 10 wt% (w/v) or 0.0001 to 5 wt% (w/v) based on the total weight of the entire composition, although not being limited thereto.

Hereinafter, the present disclosure will be described in more detail through examples, etc., but the scope and content of the present disclosure cannot be interpreted as being reduced or limited by the examples, etc. below. Furthermore, based on the disclosure of the present disclosure including the examples below, it is obvious that those skilled in the art can easily practice the present disclosure, even though specific experimental results are not presented, and such modifications and variations are also within the scope of the appended claims.

In addition, the experimental results presented below only describe representative experimental results of the above examples and comparative examples, and the effects of each of the various implementation examples of the present disclosure that are not explicitly presented below will be specifically described in the relevant section.

### <Examples>

### Example 1. Synthesis of 5-cyanoindirubin-3'-piperazinylethyloxime (CK001)

### 1-1. Synthesis of 5-cyanoindirubine, an intermediate compound

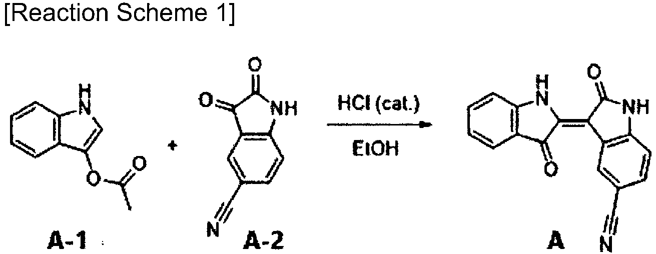

An intermediate compound of an indoline derivative (5-cyanoindirubin) was prepared according to Reaction Scheme 1. Specifically, 5-cyanoisatin (109 mg, 0.571 mmol) and 3-indoxyl acetate (1 equivalent, 100 mg, 0.571 mmol) were dissolved in ethanol (0.05 M), and then 35% hydrochloric acid was added. The mixture was heated under reflux at 80 °C for 8 hours and then cooled to room temperature. The mixture was filtered to recover a precipitate. The recovered precipitate was washed with methanol and dried under reduced pressure to recover 5-cyanoindirubin (125 mg, 74%, reddish purple solid). LC/MS (ESI, m/z) 286 [M-H]⁻; ¹H NMR (300 MHz, DMSO) δ 11.36 (s, 1H), 11.16 (s, 1H), 9.04-8.98 (m, 1H), 7.69-7.55 (m, 3H), 7.41 (d, J = 8.0 Hz, 1H), 7.08-7.00 (m, J = 8.1, 6.5, 2.7 Hz, 2H).

Example 1 is an intermediate process for synthesizing the indoline derivative according to the present disclosure, and 5-cyanoindirubin is an intermediate compound. The synthesis of the indoline derivative according to the present disclosure via the intermediate compound is advantageous in that the synthesis process is simple.

### 1-2. Synthesis of 5-cyanoindirubin-3'-oxime

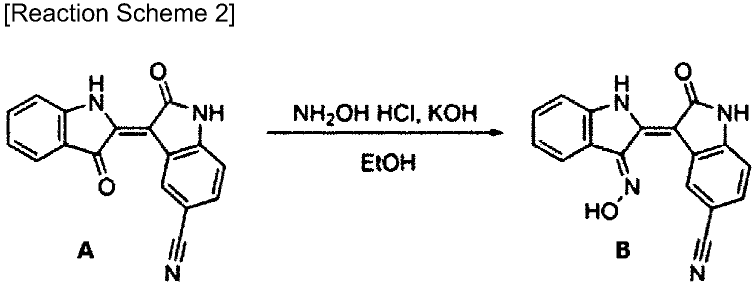

5-Cyanoindirubin-3'-oxime was synthesized according to Reaction Scheme 2. Specifically, 5-cyanoindirubin (50 mg, 0.174 mmol) was dissolved in ethanol (0.05 M), and hydroxylamine hydrochloride (7 equivalents) and potassium hydroxide (15 equivalents) were added to prepare a mixture. The mixture was heated under reflux at 80 °C for 3 hours. The heated mixture was cooled to room temperature and diluted with water (with acetic acid added). The diluted solution was filtered to recover a precipitate. The recovered precipitate was washed with water and dried under reduced pressure to obtain 5-cyanoindirubin-3'-oxime (42 mg, 75%, reddish-orange solid). LC/MS (ESI, m/z) 301 [M-H]⁻; ¹H NMR (300 MHz, DMSO) δ 13.85 (s, 1H), 11.86 (s, 1H), 11.22 (s, 1H), 8.95 (d, J = 1.6 Hz, 1H), 8.25 (d, J = 7.6 Hz, 1H), 7.56 (dd, J = 8.1, 1.7 Hz, 1H), 7.45 (qd, J = 8.4, 4.1 Hz, 2H), 7.12-7.02 (m, 2H).

### 1-3. Preparation of mixture of 5-cyanoindirubin-3'-bromoethyloxime and 5-cyanoindirubin-3'-(tosyloxy)ethyloxime

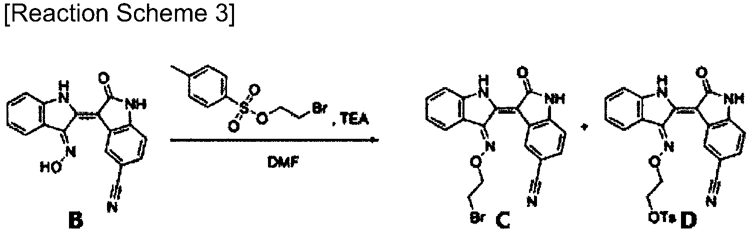

Reaction was conducted according to Reaction Scheme 3. Specifically, 5-cyanoindirubin-3'-oxime (214 mg, 0.71 mmol) was dissolved in dimethylformamide (0.15 M), and triethylamine (3 equivalents) and 2-bromoethyl-4-methylbenzenesulfonate (4 equivalents) were added dropwise to prepare a mixture. The mixture was stirred at 60 °C for 18 hours, cooled to room temperature, and then distilled under reduced pressure to remove the solvent. The mixture from which the solvent had been removed was diluted with a saturated aqueous ammonium chloride solution, and ethyl acetate was added. After recovering an organic layer from among two separated layers, sodium sulfate was added to remove moisture. The solvent was removed under reduced pressure to obtain a mixture of 5-cyanoindirubin-3'-bromoethyloxime and 5-cyanoindirubin-3'-(tosyloxy)ethyloxime (210 mg, 66%, reddish brown solid). LC/MS (ESI, m/z) 407 [M-H]⁻, 499 [M-H]⁻.

### 1-4. Synthesis of 5-cyanoindirubin-3'-piperazinylethyloxime (CK001)

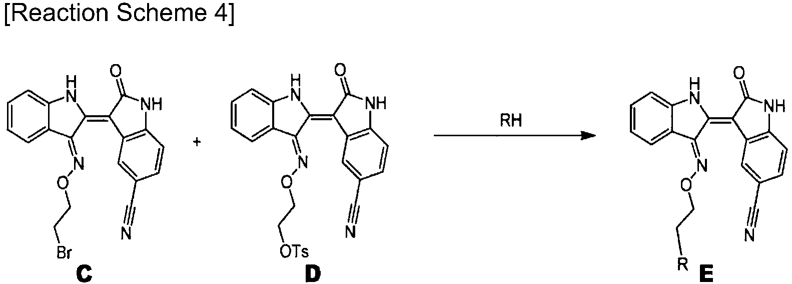

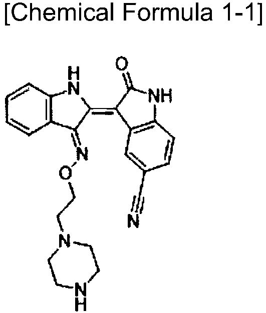

Reaction was conducted according to Reaction Scheme 4. Specifically, the mixture of 5-cyanoindirubin-3'-bromoethyloxime and 5-cyanoindirubin-3'-(tosyloxy)ethyloxime (50 mg, 0.122 mmol) was dissolved in dimethylformamide (0.15 M), and reaction was conducted by adding piperazine (5 equivalents). The reaction mixture was stirred at 50 °C for 24 hours, cooled to 0 °C, and cold water was added to generate a precipitate. After recovering the precipitate by filtering the reaction mixture, it was washed with water and dried under vacuum to obtain 5-cyanoindirubin-3'-piperazinylethyloxime (Chemical Formula 1-1, 19 mg, 34%, reddish brown solid). LC/MS (ESI, m/z) 415 [M+H]⁺, 413 [M-H]⁻; ¹H NMR (400 MHz, DMSO) δ 11.89 (s, 1H), 11.42 (s, 1H), 9.03 (s, 1H), 8.25 (d, J = 7.7 Hz, 1H), 7.67 (d, J = 8.1 Hz, 1H), 7.56 (t, J = 5.7 Hz, 2H), 7.19-7.11 (m, 2H), 4.80 (t, J = 5.8 Hz, 2H), 2.96 (t, J = 5.8 Hz, 2H), 2.76 (t, J = 4.6 Hz, 4H), 2.55 (s, 4H).

### Example 2. Synthesis of 5-cyanoindirubin-3'-morpholinylethyloxime (CK002)

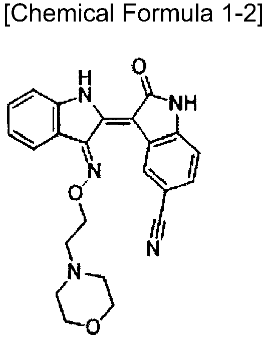

**5-cyanoindirubin-3'-morpholinylethyloxime** (Chemical Formula 1-2, 28 mg, 69%, reddish brown solid) was synthesized in the same manner as in Example 1, except that morpholine (5 equivalents) was mixed with a mixture of 5-cyanoindirubin-3'-bromoethyloxime and 5-cyanoindirubin-3'-(tosyloxy)ethyloxime (35 mg, 0.086 mmol) prepared in Example 1-3. LC/MS (ESI, m/z) 416 [M+H]⁺, 414 [M-H]⁻; ¹H NMR (400 MHz, DMSO) δ 11.76 (s, 1H), 11.25 (s, 1H), 8.92 (d, J = 4.4 Hz, 1H), 8.15 (d, J = 7.6 Hz, 1H), 7.60-7.54 (m, 1H), 7.46 (d, J = 3.4 Hz, 2H), 7.11-7.01 (m, 2H), 4.73 (t, J = 5.8 Hz, 2H), 3.61-3.54 (m, 4H), 2.92 (t, J = 5.8 Hz, 2H), 2.57-2.52 (m, 4H).

### Example 3. Synthesis of 5-cyanoindirubin-3'-thiomorpholinylethyloxime (CK003)

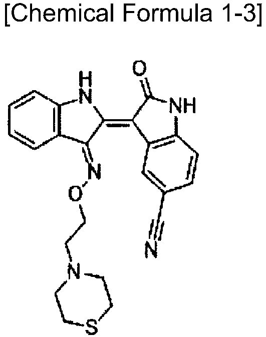

5-Cyanoindirubin-3'-thiomorpholinylethyloxime (Chemical Formula 1-3, 27 mg, 67%, reddish brown solid) was synthesized in the same manner as in Example 1, except that thiomorpholine (5 equivalents) was mixed with the mixture of 5-cyanoindirubin-3'-bromoethyloxime and 5-cyanoindirubin-3'-(tosyloxy)ethyloxime (35 mg, 0.086 mmol) prepared in Example 1-3. LC/MS (ESI, m/z) 432 [M+H]⁺, 430 [M-H]⁻; ¹H NMR (400 MHz, DMSO) δ 11.77 (s, 1H), 11.26 (s, 1H), 8.93 (dd, J = 6.7, 1.5 Hz, 1H), 8.17 (t, J = 10.0 Hz, 1H), 7.57 (dd, J = 8.1, 1.6 Hz, 1H), 7.47 (dd, J = 6.0, 2.7 Hz, 2H), 7.11-7.01 (m, 2H), 4.71 (t, J = 5.9 Hz, 2H), 2.97 (t, J = 5.9 Hz, 2H), 2.87-2.76 (m, 4H), 2.63-2.55 (m, 4H).

### Example 4. Synthesis of 5-cyanoindirubin-3'-(4-methylpiperazinyl)ethyloxime (CK004)

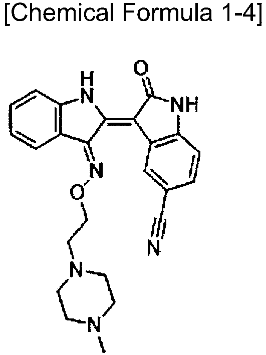

5-Cyanoindirubin-3'-(4-methylpiperazinyl)ethyloxime (Chemical Formula 1-4, 27 mg, 65%, reddish brown solid) was synthesized in the same manner as in Example 1, except that N-methylpiperazine (5 equivalents) was mixed with the mixture of 5-cyanoindirubin-3'-bromoethyloxime and 5-cyanoindirubin-3'-(tosyloxy)ethyloxime (35 mg, 0.086 mmol) prepared in Example 1-3. LC/MS (ESI, m/z) 429 [M+H]⁺, 427 [M-H]⁻; ¹H NMR (400 MHz, DMSO) δ 11.77 (s, 1H), 11.26 (s, 1H), 8.92 (d, J = 0.8 Hz, 1H), 8.16 (d, J = 7.7 Hz, 1H), 7.57 (dd, J = 8.1, 1.5 Hz, 1H), 7.46 (d, J = 4.0 Hz, 2H), 7.07 (ddd, J = 17.9, 10.3, 5.8 Hz, 2H), 4.71 (t, J = 5.9 Hz, 2H), 2.91 (t, J = 5.9 Hz, 2H), 2.55 (s, 4H), 2.31 (s, 4H), 2.13 (s, 3H).

### Example 5. Synthesis of 5-cyanoindirubin-3'-piperidinylethyloxime (CK005)

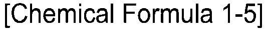

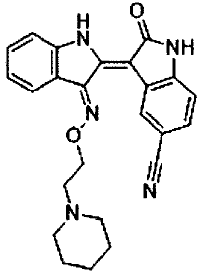

5-Cyanoindirubin-3'-piperidinylethyloxime (Chemical Formula 1-5, 33 mg, 60%, reddish brown solid) was synthesized in the same manner as in Example 1, except that piperidine (5 equivalents) was mixed with the mixture of 5-cyanoindirubin-3'-bromoethyloxime and 5-cyanoindirubin-3'-(tosyloxy)ethyloxime (50 mg, 0.122 mmol) prepared in Example 1-3. LC/MS (ESI, m/z) 414 [M+H]⁺; ¹H NMR (400 MHz, DMF) δ 11.97 (s, 1H), 11.27 (s, 1H), 9.07 (d, J = 1.3 Hz, 1H), 8.29 (d, J = 7.6 Hz, 1H), 7.69-7.50 (m, 3H), 7.27-7.11 (m, 2H), 4.84 (t, J = 5.9 Hz, 2H), 2.98 (t, J = 5.9 Hz, 2H), 2.68-2.46 (m, 4H), 1.55 (dt, J = 10.8, 5.5 Hz, 4H), 1.46-1.37 (m, J = 16.7 Hz, 2H).

### Example 6. Synthesis of 5-cyanoindirubin-3'-pyrrolidinylethyloxime (CK006)

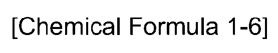

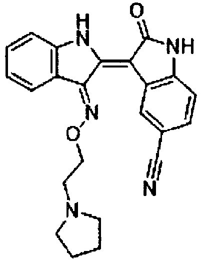

5-Cyanoindirubin-3'-pyrrolidinylethyloxime (Chemical Formula 1-6, 17 mg, 31%, reddish brown solid) was synthesized in the same manner as in Example 1, except that pyrrolidine (5 equivalents) was mixed with the mixture of 5-cyanoindirubin-3'-bromoethyloxime and 5-cyanoindirubin-3'-(tosyloxy)ethyloxime (50 mg, 0.122 mmol) prepared in Example 1-3. LC/MS (ESI, m/z) 400 [M+H]⁺; ¹H NMR (400 MHz, DMSO) δ 11.78 (s, 1H), 11.30 (s, 1H), 8.94 (d, J = 1.2 Hz, 1H), 8.15 (d, J = 7.7 Hz, 1H), 7.57 (dd, J = 8.1, 1.5 Hz, 1H), 7.49-7.44 (m, 2H), 7.11-7.06 (m, 1H), 7.04 (d, J = 8.1 Hz, 1H), 4.71 (t, J = 5.9 Hz, 2H), 3.03 (t, J = 5.9 Hz, 2H), 2.62-2.54 (m, 4H), 1.68 (dd, J = 6.5, 3.2 Hz, 4H).

### Example 7. Synthesis of 5-cyanoindirubin-3'-piperidinylpropyloxime (CK007)

### 7-1. Synthesis of 5-cyanoindirubin-3-bromopropyloxime

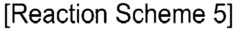

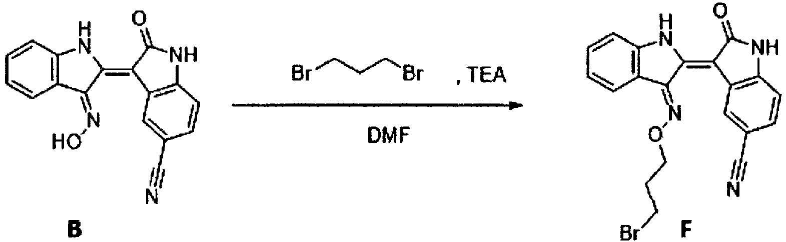

After dissolving 5-cyanoindirubin-3'-oxime (200 mg, 0.71 mmol) represented by Chemical Formula B prepared according to Reaction Scheme 2 of Example 1 in dimethylformamide (0.15 M), triethylamine (3 equivalents) and 1,3-dibromopropane (4 equivalents) were added dropwise to prepare a mixture. The mixture was stirred at 60°C for 18 hours, cooled to room temperature, and the solvent was removed under reduced pressure. The mixture from which the solvent had been removed was diluted with a saturated aqueous ammonium chloride solution and then extracted with ethyl acetate. Sodium sulfate was added to the organic layer to remove moisture, and the solvent was removed under reduced pressure to synthesize 5-cyanoindirubin-3'-bromopropyloxime (Chemical Formula F, 176 mg, 63%, red solid). LC/MS (ESI, m/z) 421 [M-H]⁻; ¹H NMR (400 MHz, DMF) δ 11.97 (s, 1H), 11.25 (s, 1H), 9.07 (s, 1H), 8.26 (d, J = 7.6 Hz, 1H), 7.66 (dd, J = 8.1, 1.4 Hz, 1H), 7.57 (dt, J = 15.0, 7.6 Hz, 2H), 7.22-7.15 (m, 2H), 4.90 (t, J = 6.1 Hz, 2H), 3.85 (t, J = 6.6 Hz, 2H), 2.63 (p, J = 6.3 Hz, 2H).

### 7-2. Synthesis of 5-cyanoindirubin-3'-piperidinylpropyloxime derivative (CK007)

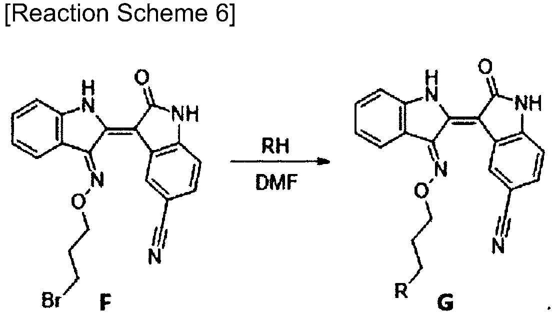

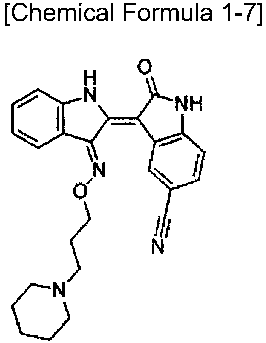

5-Cyanoindirubin-3'-bromopropyloxime (50 mg, 0.118 mmol) was dissolved in dimethylformamide (0.15 M). After adding piperidine (5 equivalents) to the solution and stirring at 50 °C for 24 hours, the mixture was cooled to 0 °C to prepare a reaction solution. After adding cold water to the reaction solution, a precipitate was recovered by filtration. The recovered precipitate was washed with water and dried under vacuum to synthesize **5-cyanoindirubin-3'-piperidinylpropyloxime** (Chemical Formula 1-7, 47 mg, 81%, red solid). LC/MS (ESI, m/z) 428 [M+H]⁺, 426 [M-H]⁻; ¹H NMR (400 MHz, DMF) δ 11.96 (s, 1H), 11.24 (s, 1H), 9.08 (d, J = 1.2 Hz, 1H), 8.25 (d, J = 7.7 Hz, 1H), 7.63 (dd, J = 8.1, 1.6 Hz, 1H), 7.55 (q, J = 8.0 Hz, 2H), 7.22-7.13 (m, 2H), 4.79 (t, J = 6.5 Hz, 2H), 2.57 (t, J = 7.0 Hz, 2H), 2.51-2.30 (m, 4H), 2.25-2.17 (m, 2H), 1.54 (dt, J = 10.7, 5.5 Hz, 4H), 1.44-1.36 (m, 2H).

### Example 8. Synthesis of 5-cyanoindirubin-3'-pyrrolidinylpropyloxime (CK008)

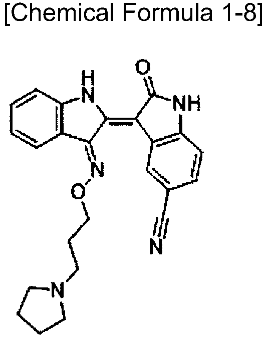

5-Cyanoindirubin-3'-pyrrolidinylpropyloxime (Chemical Formula 1-8, 43 mg, 75%, red solid) was synthesized in the same manner as in Example 7-2, except that pyrrolidine (5 equivalents) was mixed with the 5-cyanoindirubin-3'-bromopropyloxime (50 mg, 0.118 mmol) prepared in Example 7-1. LC/MS (ESI, m/z) 414 [M+H]⁺, 412 [M-H]⁻; ¹H NMR (400 MHz, DMSO) δ 11.79 (s, 1H), 11.29 (s, 1H), 8.96 (d, J = 1.1 Hz, 1H), 8.13 (d, J = 7.6 Hz, 1H), 7.58 (dd, J = 8.1, 1.4 Hz, 1H), 7.53-7.45 (m, 2H), 7.12-7.07 (m, 1H), 7.04 (d, J = 8.1 Hz, 1H), 4.69 (t, J = 6.4 Hz, 2H), 2.61 (t, J = 7.0 Hz, 2H), 2.47-2.41 (m, 4H), 2.15-2.07 (m, 2H), 1.72-1.62 (m, 4H).

### Example 9. Synthesis of 5-cyanoindirubin-3'-piperazinylpropyloxime (CK009)

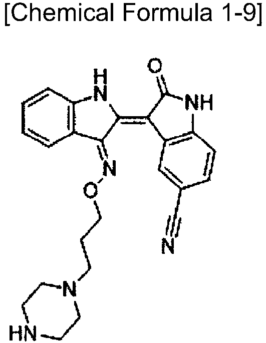

5-Cyanoindirubin-3'-piperazinylpropyloxime (Chemical Formula 1-9, 29 mg, 82%, red solid) was synthesized in the same manner as in Example 7-2, except that piperazine (5 equivalents) was mixed with the 5-cyanoindirubin-3'-bromopropyloxime (30 mg, 0.071 mmol) prepared in Example 7-1. LC/MS (ESI, m/z) 429 [M+H]; ¹H NMR (300 MHz, DMSO) δ 12.04-11.06 (m, 2H), 8.96 (d, J = 1.4 Hz, 1H), 8.13 (d, J = 7.6 Hz, 1H), 7.57 (dd, J = 8.0, 1.6 Hz, 1H), 7.51-7.44 (m, 2H), 7.13-7.02 (m, 2H), 4.67 (t, J = 6.5 Hz, 2H), 2.73-2.57 (m, 4H), 2.46 (t, J = 7.1 Hz, 2H), 2.41-2.20 (m, 4H), 2.15-2.04 (m, 2H).

### Example 10. Synthesis of 5-cyanoindirubin-3'-morpholinylpropyloxime (CK010)

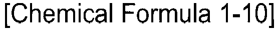

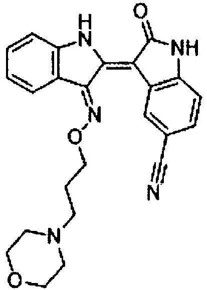

5-Cyanoindirubin-3'-morpholinylpropyloxime (Chemical Formula 1-10, 21 mg, 70%, red solid) was synthesized in the same manner as in Example 7-2, except that morpholine (5 equivalents) was mixed with the 5-cyanoindirubin-3'-bromopropyloxime (25 mg, 0.059 mmol) prepared in Example 7-1. LC/MS (ESI, m/z) 430 [M+H]⁺; ¹H NMR (300 MHz, DMSO) δ 11.76 (s, 1H), 11.26 (s, 1H), 8.98 (d, J = 1.6 Hz, 1H), 8.14 (d, J = 7.6 Hz, 1H), 7.59 (dd, J = 8.1, 1.7 Hz, 1H), 7.47 (m, 2H), 7.09 (m, 2H), 4.69 (t, J = 6.5 Hz, 2H), 3.56 (m, 4H), 2.53 (t, J = 6.1 Hz, 2H), 2.38 (m, 4H), 2.12 (dt, J = 13.5, 6.6 Hz, 2H).

### Example 11. Synthesis of 5-cyanoindirubin-3'-(4-methylpiperazinyl)propyloxime (CK011)

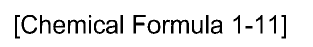

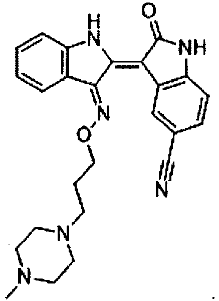

5-Cyanoindirubin-3'-(4-methylpiperazinyl)propyloxime (Chemical Formula 1-11, 30 mg, 89%, red solid) was synthesized in the same manner as in Example 7-2, except that N-methylpiperazine (5 equivalents) was mixed with the 5-cyanoindirubin-3'-bromopropyloxime (25 mg, 0.059 mmol) prepared in Example 7-1. LC/MS (ESI, m/z) 443 [M+H]⁺; ¹H NMR (400 MHz, DMF) δ 12.15 (s, 1H), 11.42 (s, 1H), 9.26 (d, J = 1.5 Hz, 1H), 8.42 (d, J = 7.6 Hz, 1H), 7.82 (dd, J = 8.1, 1.6 Hz, 1H), 7.76-7.69 (m, 2H), 7.38-7.32 (m, 2H), 4.97 (t, J = 6.5 Hz, 2H), 2.77 (t, J = 7.1 Hz, 2H), 2.74-2.40 (m, 8H), 2.41-2.37 (m, 2H), 2.34 (s, 3H).

### Example 12. Synthesis of 5-cyanoindirubin-3'-thiomorpholinylpropyloxime (CK012)

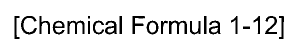

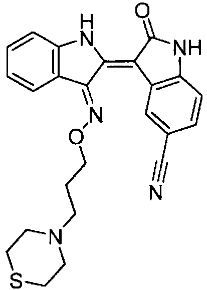

5-Cyanoindirubin-3'-thiomorpholinylpropyloxime (Chemical Formula 1-12, 27 mg, 83%, red solid) was synthesized in the same manner as in Example 7-2, except that thiomorpholine (5 equivalents) was mixed with the 5-cyanoindirubin-3'-bromopropyloxime (25 mg, 0.059 mmol) prepared in Example 7-1. LC/MS (ESI, m/z) 446 [M+H]⁺; ¹H NMR (300 MHz, DMSO) δ 11.78 (s, 1H), 11.26 (s, 1H), 8.97 (d, J = 1.3 Hz, 1H), 8.13 (d, J = 7.6 Hz, 1H), 7.58 (dd, J = 8.1, 1.7 Hz, 1H), 7.51-7.44 (m, 2H), 7.13-7.03 (m, 2H), 4.67 (t, J = 6.5 Hz, 2H), 2.70-2.62 (m, 4H), 2.61-2.56 (m, 4H), 2.54 (t, J = 7.9 Hz, 2H), 2.15-2.04 (m, 2H).

### Example 13. Synthesis of 5-cyanoindirubin-3'-(1,1-dioxothiomorpholinyl)ethyloxime (CK013)

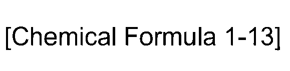

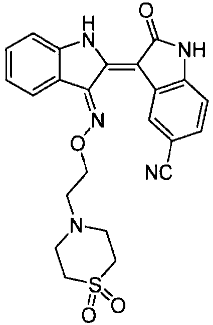

The mixture of 5-cyanoindirubin-3'-bromoethyloxime and 5-cyanoindirubin-3'-(tosyloxy)ethyloxime (0.122 mmol) prepared in Example 1-3 was dissolved in N-methyl-2-pyrrolidone (0.15 M), and thiomorpholine 1,1-dioxide (2 equivalents), potassium carbonate (10 equivalents), and potassium iodide (0.1 equivalent) were added to cause a reaction. The reaction solution was stirred at 50 °C for 12 hours, cooled to 0 °C, and cold water was added to generate a precipitate. After recovering the precipitate through filtration, it was washed with water and dried under vacuum to obtain 5-cyanoindirubin-3'-(1,1-dioxothiomorpholinyl)ethyloxime (Formula 1-13, 150 mg, 40%, red solid). LC/MS (ESI, m/z) 464 [M+H]⁺; ¹H NMR (400 MHz, DMSO) δ 11.77 (s, 1H), 11.27 (s, 1H), 8.92 (s, 1H), 8.16 (d, J = 7.6 Hz, 1H), 7.59-7.47 (m, 1H), 7.46 (m, 2H), 7.10-7.03 (m, 2H), 4.73 (t, J = 5.6 Hz, 2H), 3.16 (t, J = 5.4 Hz, 2H), 3.09 (m, 8H).

### Example 14. Synthesis of 5-cyanoindirubin-3'-(1-oxothiomorpholinyl)ethyloxime hydrochloride (CK014)

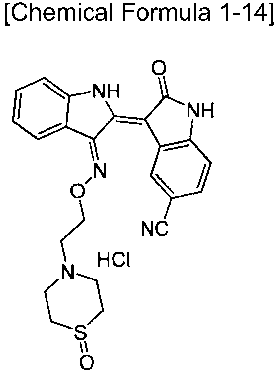

5-Cyanoindirubin-3'-(1-oxothiomorpholinyl)ethyloxime was synthesized in the same manner as in Example 13 (Chemical Formula 1-13), except that thiomorpholine 1-oxide (2 equivalents) was mixed with the mixture of 5-cyanoindirubin-3'-bromoethyloxime and 5-cyanoindirubin-3'-(tosyloxy)ethyloxime (0.086 mmol) prepared in Example 1-3.

Specifically, the product synthesized through the process was dissolved in THF and, after adding HCI/EtOAc (10 equivalents), the mixture was stirred at room temperature for 12 hours. Afterwards, the mixture was concentrated under reduced pressure to obtain 5-cyanoindirubin-3'-(1-oxothiomorpholinyl)ethyloxime hydrochloride (Chemical Formula 1-14, 500 mg, 60%, red solid). LC/MS (ESI, m/z) 448 [M+H]⁺; ¹H NMR (400 MHz, DMSO) δ 11.81 (s, 1H), 11.35 (s, 1H), 10.45-10.38 (m, 1H), 8.91 (s, 1H), 8.26 (m, 1H), 7.62 (d, J = 8.4 Hz, 1H), 7.51-7.50 (m, 2H), 7.12-7.06 (m, 2H), 5.04 (m, 2H), 3.94-3.91 (m, 5H), 3.37-3.00 (m, 5H).

### Example 15. Synthesis of 5-cyanoindirubin-3'-(2-prolinyl)ethyloxime (CK015)

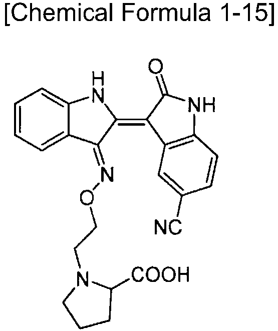

The mixture of 5-cyanoindirubin-3'-bromoethyloxime and 5-cyanoindirubin-3'-(tosyloxy)ethyloxime (0.122 mmol) prepared in Example 1-3 was dissolved in dimethylformamide (0.15 M), and tert-butyl prolinate (2 equivalents), potassium carbonate (10 equivalents), and potassium iodide (0.1 equivalent) were added to cause a reaction. The reaction solution was stirred at 50 °C for 12 hours, cooled to 0 °C, and cold water was added to generate a precipitate. After recovering the precipitate through filtration, it was washed with water, and dried under vacuum to obtain tert-butyl (2-((((2Z,3E)-5'-cyano-2'-oxo-[2,3'-biindolinylidene]-3-ylidene)amino)oxy)ethyl)prolinate. The obtained product was dissolved in dichloromethane (0.4 M) and trifluoroacetic acid (10 equivalents) was added at 0 °C. The mixture was stirred at room temperature for 2 hours, and then distilled under reduced pressure. After dissolving the mixture in dichloromethane and 1 N hydrochloric acid, an organic layer was separated and extracted. After drying over anhydrous sodium sulfate, the residue was concentrated under reduced pressure and purified by column chromatography to obtain 5-cyanoindirubin-3'-(2-prolinyl)ethyloxime (Formula 1-15, 150 mg, 39%, red solid). LC/MS (ESI, m/z) 444 [M+H]⁺; ¹H NMR (400 MHz, DMSO) δ 11.76 (s, 1H), 11.35 (s, 1H), 8.85 (s, 1H), 8.23 (d, J = 7.6 Hz, 1H), 7.57 (d, J = 8.4 Hz, 1H), 7.47-7.46 (m, 2H), 7.09-7.05 (m, 2H), 4.93 (m, 2H), 4.31-4.29 (m, 1H), 3.92 (m, 1H), 3.73 (m, 1H), 3.27-3.20 (m, 2H), 2.40-2.37 (m, 1H), 2.03-1.99 (m, 2H), 1.88 (m, 1H).

### <Test Examples>

### Test Example 1. Analysis of CXXC5-Dvl interaction inhibition activity of indoline derivatives of Examples 1 to 15

It was investigated whether the indoline derivative according to the present disclosure specifically inhibits CXXC5-Dvl interaction. To this end, the following experiments is conducted to evaluate the CXXC5-Dvl binding inhibition effect of the indoline compounds prepared in Examples 1 to 15.

5 µg/mL of the purified PDZ domain ofDvl (dishevelled) protein was attached to the bottom of a 96-well black immunoplate and left in a chamber at 4 °C for one day. The next day, each well was washed with PBS and 10 µM PTD-DBM-FITC was added. After incubation at room temperature for 3 hours, each well was washed with PBS. Each well was treated with the indoline derivatives at various concentrations (100, 10, 1, 0.1, 0.01, and 0.001 µM) in 100 µL of PBS. After washing with a buffer, fluorescence was measured by FITC using a microplate reader. A competition binding curve showing the degree of CXXC5-Dvl PPI inhibition was constructed using the GraphPad Prism software, and the IC₅₀ value was calculated.

The experimental was conducted using a self-developed screening system, and the CXXC5-Dvl binding inhibition effect of the indoline derivatives of Examples 1 to 15 was analyzed using an existing indirubin compound (Chemical Formula 1-16), 6-chloro-5-methylindirubin-3'-(4-methylpiperazinyl)ethyloxime (Chemical Formula 1-17; CK090), and the intermediate compound 5-cyanoindirubin as controls.

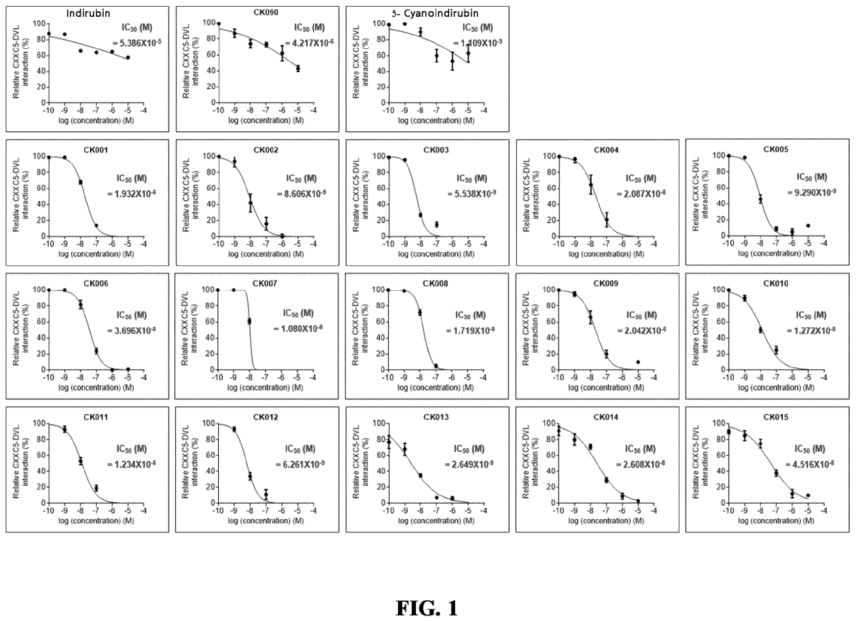

FIG. 1 shows a result of analyzing the concentration-dependent CXXC5-Dvl binding inhibition activity of the indoline derivatives of Examples 1 to 15, 6-chloro-5-methylindirubin-3'-(4-methylpiperazinyl)ethyloxime (CK090), an intermediate compound (5-cyanoindirubin), and an indirubin compound.

As shown in FIG. 1, all of the indoline derivatives of Examples 1 to 15 showed significantly superior CXXC5-Dvl binding inhibition effect as compared to the indirubin compound (indirubin) (IC₅₀ (M) = 5.386x10⁻⁵), 6-chloro-5-methylindirubin-3'-(4-methylpiperazinyl)ethyloxime (CK090) (IC₅₀ (M) = 4.217x10⁻⁶) and the intermediate compound (5-cyanoindirubin) (IC₅₀ (M) = 1.109x10⁻⁵) with the following IC₅₀ values (1.932x10⁻⁸, 8.606x10⁻⁹, 5.538x10⁻⁹, 2.087x10⁻⁸, 9.290x10⁻⁹, 3.696x10⁻⁸, 1.080x10⁻⁸, 1.719×10⁻⁸, 2.042×10⁻⁸, 1.272×10⁻⁸, 1.234×10⁻⁸, 6.261×10⁻⁹, 2.649×10⁻⁹, 2.608×10⁻⁸, and 4.516×10⁻⁸ in order).

In other words, unlike conventional indirubin compounds, 6-chloro-5-methylindirubin-3'-(4-methylpiperazinyl)ethyloxime, and intermediate compounds, the indolin derivatives according to Examples 1 to 15 exhibit a direct inhibitory effect on the binding between CXXC5 and Dvl, thereby demonstrating a statistically significant and pronounced inhibitory activity against the CXXC5-Dvl interaction..

Therefore, it can be seen that the indoline derivatives prepared in Examples 1 to 15 of the present disclosure can be used as useful compositions for inhibiting CXXC5-Dvl interaction.

### Test Example 2. Analysis of ability to inhibit adipocyte differentiation and lipid accumulation of indoline derivatives of Examples 1 to 15

It was investigated whether the indoline derivative inhibits of the present disclosure inhibit adipocyte differentiation and lipid accumulation. To this end, the following experiment was conducted to evaluate the adipocyte differentiation inhibitory effect of the indoline compounds prepared in Examples 1 to 15.

3T3-L1 cells were seeded in a 6-well plate at a density of 8x10⁻⁴ cells per well. The cells were grown to 100% confluency in Dulbecco's modified Eagle's medium (DMEM) supplemented with 10% bovine calf serum (BCS; GIBCO). Two days after reaching 100% confluency, differentiation was induced with or without treatment with the indoline compound at a concentration of 1 µM in DMEM containing 10% fetal bovine serum (FBS; GIBCO) and MDI (520 µM methylisobutylxanthine (IBMX; Sigma-Aldrich), 1 µM dexamethasone (Sigma-Aldrich), and 5 µM insulin (GIBCO)). On day 3, the medium was replaced with DMEM containing 10% FBS and 5 µM insulin with or without the indoline compound, and then with a fresh identical medium every 2 days. After differentiation for 8 days, the culture medium was removed, and the cells were washed with PBS and then fixed with 10% formaldehyde for 1 hour. After removing the 10% formaldehyde, the cells were washed thoroughly with distilled water. After that, 60% isopropanol was added and immediately then removed. After washing with distilled water, lipid droplets were stained with an Oil Red O dye solution for 20 minutes and then washed with distilled water. The cells were counterstained with Mayer's hematoxylin. Oil Red O images were visualized using a bright-field microscope (Nikon TE-2000U). To quantify the accumulated lipids, Oil Red O was eluted by adding isopropanol, and absorbance was measured spectrophotometrically at 544 nm using a multi-plate reader.

As A3051 (KY19382) and A3334, self-developed and synthesized materials were used. As BIO, or GSK 3 Inhibitor IX, also known as 6-bromoindirubin-3'-oxime, HY-10580 was purchased commercially.

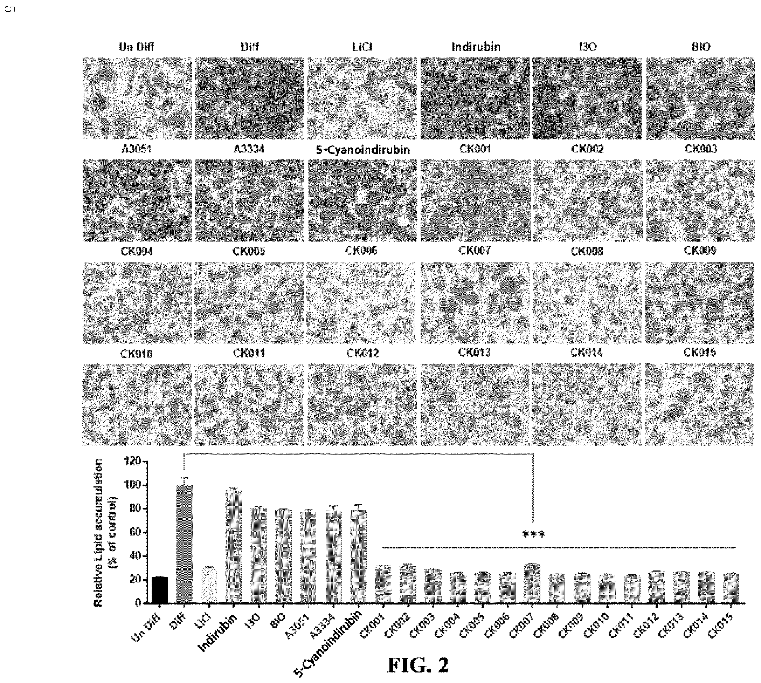

FIG. 2 shows a result of comparing the Oil Red O staining images and lipid accumulation amounts of the indoline derivatives of Examples 1 to 15 as compared to LiCl used as a positive control of adipocyte differentiation, and an existing indirubin compound, I3O, BIO, A3051, A3334, and 5-cyanoindirubin as an intermediate compound, as controls. In FIG. 2, the undifferentiated group (Un Diff) refers to undifferentiated adipocytes, or preadipocytes that had not been induced to differentiate, and the differentiated group (Diff) refers to fully differentiated adipocytes.

As shown in FIG. 2, the indoline derivatives of Examples 1 to 15 showed significantly superior effect of inhibiting lipid accumulation (32.2%, 32.3%, 28.9%, 26.0%, 26.2%, 25.8%, 33.6%, 25.1%, 25.4%, 24.3%, 24.1%, 27.4%, 26.6%, 26.8%, and 24.8% in order) as compared to the indirubin compound (indirubin) (lipid accumulation rate = 96.4%), I3O (lipid accumulation rate = 80.6%), BIO (lipid accumulation rate = 79.4%), A3051 (lipid accumulation rate = 77.4%), A3334 (lipid accumulation rate = 78.7%), or the intermediate compound (5-cyanoindirubin) (lipid accumulation rate = 79.0%).

In other words, it can be seen that the indoline derivatives according to Examples 1 to 15 achieve a significantly more remarkable anti-obesity effect as compared to the conventional indirubin compound, I3O, BIO, A3051, A3334 and the intermediate compound.

Therefore, it can be seen that the indoline derivatives prepared in Examples 1 to 15 of the present disclosure can be used as useful compositions for treating various metabolic diseases including obesity through inhibition of adipocyte differentiation.

### Test Example 3. Analysis of efficacy of indoline derivatives for metabolic diseases

### 3-1. Animal experiment

Five-week-old wild-type male C57BL/6N mice were purchased from Orient Bio Co., Ltd. They were allowed to acclimate to the laboratory environment for one week. Mice weighing 18-20 g on average were used in the experiment. To ensure ethical and scientific validity and efficient management of animal experiments, the experiment was conducted with the approval of the Institutional Review Board (IACUC-A-202205-1472-01) of Severance Hospital, Yonsei University College of Medicine.

The mice were divided into a normal group (NCD) and a high-fat diet group (HFD). The normal group (NCD) was fed a normal chow diet (NCD, Teklad Global 18% protein rodent diet), and the high-fat diet group (HFD) was fed a high-fat diet (HFD, D12492) in which fat accounted for 60% of the total calories, for 8 weeks.

Mice induced with metabolic dysfunction-associated steatohepatitis (MASH) (hereinafter, referred to as MASH or MASH group) were prepared by administering CCl₄ intraperitoneally twice a week for 4 weeks after feeding the high-fat diet (HFD) for 8 weeks. The CCl₄ was diluted with corn oil at a ratio of 1:19 and injected at a dosage of 2 mL/kg. The normal group (NCD) was administered the same volume of physiological saline.

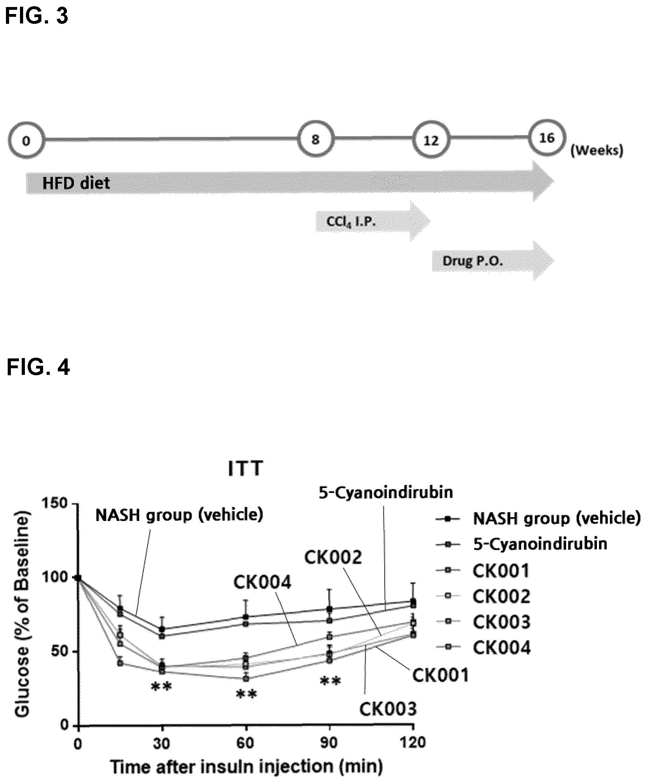

The animals were divided into 7 groups, each with 10 animals. The specific experimental model is as follows, and the experimental design is shown in FIG. 3.

**[Table 1]**

| Category | Experiment method |
|---|---|
| Normal group | Normal chow diet for 8 weeks + physiological saline 2 mL/kg |
| (NCD) | intraperitoneally twice a week for 4 weeks + buffer solution 25 mg/kg/day orally for 4 weeks |
| MASH group (vehicle) | High-fat diet for 8 weeks + CCl₄ 2 mL/kg intraperitoneally twice a week for 4 weeks + buffer solution 25 mg/kg/day orally for 4 weeks |
| Test group 1 | High-fat diet for 8 weeks + CCl₄ 2 mL/kg intraperitoneally twice a week for 4 weeks + intermediate compound of Example 1-1 25 mg/kg/day orally for 4 weeks |
| Test group 2 (CK001) | High-fat diet for 8 weeks + CCl₄ 2 mL/kg intraperitoneally twice a week for 4 weeks + 5-cyanoindirubin-3'-piperazinylethyloxime of Example 1, 25 mg/kg/day orally for 4 weeks |
| Test group 3 (CK002) | High-fat diet for 8 weeks + CCl₄ 2 mL/kg intraperitoneally twice a week for 4 weeks + 5-cyanoindirubin-3'-morpholinylethyloxime of Example 2, 25 mg/kg/day orally for 4 weeks |
| Test group 4 (CK003) | High-fat diet for 8 weeks + CCl₄ 2 mL/kg intraperitoneally twice a week for 4 weeks + 5-cyanoindirubin-3'-thiomorpholinylethyloxime of Example 3, 25 mg/kg/day orally for 4 weeks |
| Test group 5 (CK004) | High-fat diet for 8 weeks + CCl₄ 2 mL/kg intraperitoneally twice a week for 4 weeks + 5-cyanoindirubin-3'-(4-methylpiperazinyl)ethyloxime of Example 4, 25 mg/kg/day orallyfor 4 weeks |

### 3-2. Analysis of insulin sensitivity in animal model of nonalcoholic fatty liver disease

In Test Example 1, it was confirmed that the indoline derivatives of Examples 1 to 15 have inhibition activity against the CXXC5-Dvl interaction. It was investigated whether the inhibition activity leads to a therapeutic effect on metabolic diseases such as nonalcoholic fatty liver disease.

First, an insulin tolerance test (ITT) was performed. Specifically, the 7 groups were fasted for 4 hours, and then human insulin was injected at a dose of 0.75 IU/kg. After collecting tail blood samples from each group at intervals of 0, 15, 30, 60, 90, and 120 minutes, blood glucose levels were measured using a One Touch Ultra blood glucose meter (LifeScan).

FIG. 4 shows the insulin tolerance test (ITT) result for the MASH group (vehicle) and the test groups 1 to 5. It was confirmed that the insulin resistance of the test groups 2 to 5, to which the indoline derivatives of Examples 1 to 4 were administered orally, was significantly improved as compared to the MASH group (vehicle) or the test group 1.

### 3-3. Histological analysis in animal model of nonalcoholic fatty liver disease

After preparing the 7 groups, the animals were sacrificed using carbon dioxide and the liver tissues were extracted. The extracted tissues were fixed in 4% neutral paraformaldehyde and embedded in paraffin to prepare sections. The paraffin sections were cut to 4 µm thickness and H&E staining was performed. Twenty randomly selected microscopic fields were photographed and analyzed using a Nikon bright-field optical microscope (Nikon TE-2000U).

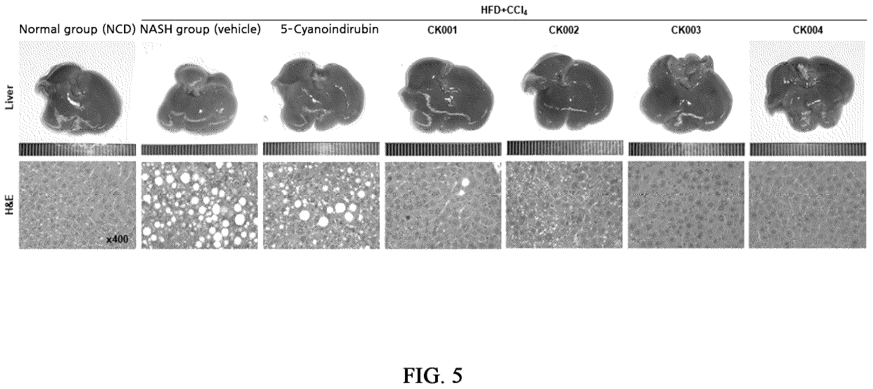

FIG. 5 shows the result of histological analysis of morphologically observing the liver tissues extracted from the normal group (NCD), the MASH group (vehicle), and the test groups 1 to 5, and staining the same with hematoxylin and eosin (H&E).

As shown in FIG. 5, when the liver tissue was extracted from each group and observed morphologically, the liver tissue of the MASH group (vehicle) turned yellow as compared to the liver tissue of the normal group (NCD). This means that fatty liver was formed by CCl₄.

It was found that in the test group 1, to which 5-cyanoindirubin, i.e., the intermediate compound of Example 1-1, was administered orally, fatty liver was not alleviated significantly as compared to the MASH group (vehicle).

On the other hand, the liver tissues of the test groups 2 to 5, to which indoline derivatives of Examples 1 to 4 were administered orally, were confirmed to exhibit a reddish brown color similar to the liver tissue of the normal group (NCD), indicating that fatty liver induced by MASH was effectively treated and restored to a normal level.

As a result of staining the liver tissue extracted from each group with H&E and observing under a microscope (bottom of FIG. 5), the liver tissue of the normal group (NCD) showed almost no lipid accumulation or inflammatory response caused thereby, whereas the liver tissue of the MASH group (vehicle) showed clear steatosis.

In the liver tissue of the test group 1, to which 5-cyanoindirubin, or the intermediate compound of Example 1-1, was administered orally, steatosis was also confirmed clearly. On the other hand, the liver tissues of the test groups 2 to 5, to which the indoline derivatives of Examples 1 to 4 were administered orally, showed reduced lipid droplets and recovery to a level similar to that of the normal group (NCD).

### 3-4. Analysis of MAFLD (metabolic dysfunction-associated fatty liver disease) activity score in animal models of metabolic dysfunction-associated steatohepatitis

After preparing the 7 groups, the animals were sacrificed using carbon dioxide and the liver tissues were extracted. After staining the liver tissues with H&E, scores were calculated using the MAFLD activity scoring system. The MAFLD activity scoring system proposed in 2005 classifies disease based on the following three criteria (Kleiner DE et al., 2005).
1) Steatosis (0: 5% or less, 1: 5-33%, 2: 33-66%, 3: 66% or more of infiltration is observed in hepatocytes).
2) Intralobular inflammation (0: no foci, 1: 4 foci per 200x field).
3) Balloon expansion (0: none, 1: few, 2: many cells/prominent ballooning).

If the total score of the three criteria above is 5 points or higher, it is judged as MASH, if it is 3 points or lower, it is judged as not MASH, and if it is 3 or 4 points, it is judged as borderline.

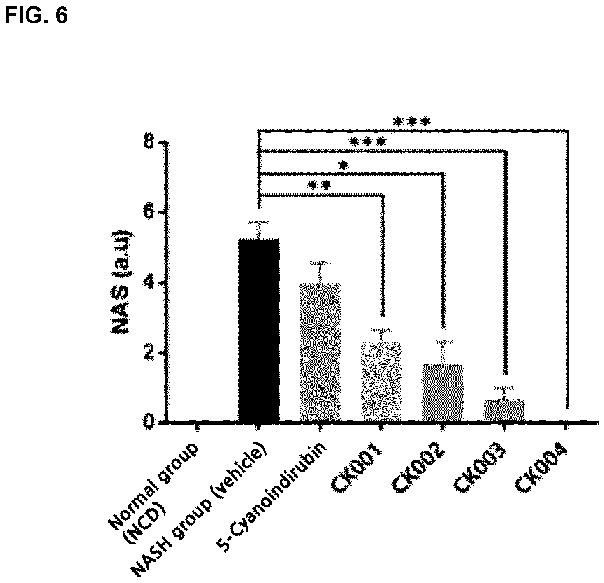

FIG. 6 shows a result of extracting liver tissues from the normal group (NCD), the MASH group (vehicle), and the test groups 1 to 5, and evaluating the MAFLD activity score to confirm histological changes.

As shown in FIG. 6, as a result of calculating the scores of the groups using the MAFLD activity scoring system, the score of the MASH group (vehicle) was 5 points or higher, confirming that nonalcoholic fatty liver disease was induced properly. The test group 1 did not show significant difference in the score as compared to the MASH group (vehicle). On the other hand, the test groups 2 to 5 showed significantly improved scores of lower than 3 points as compared to the MASH group (vehicle). Therefore, it can be seen that the indoline derivatives of Examples 1 to 4 have CXXC5-Dvl interaction inhibition activity, and thus can be usefully applied as therapeutic agents for metabolic diseases.

### 3-5. Analysis of triglyceride accumulation in liver of animal model of metabolic dysfunction-associated steatohepatitis (MASH; nonalcoholic fatty liver disease/steatohepatitis)

### Oil Red O staining

It was investigated whether the accumulation of triglycerides in the liver is suppressed effectively. For this purpose, liver tissue was extracted from each group, and it was prepared into 10-µm thick cryosections, which were then stained with Oil Red O. Details are as follows. After preparing the 7 groups, the animals were euthanized using carbon dioxide and the liver tissues were extracted. A certain area of the extracted liver tissue was cryosectioned to 10 µm thickness, fixed in 4% neutral paraformaldehyde for 20 minutes at room temperature, and washed with 70% isopropanol (Duksan Pure Chemicals). After staining with the Oil Red O (Sigma-Aldrich) reagent for 20 minutes, the sections were washed thoroughly three times with distilled water. The tissue was counterstained with Mayer's hematoxylin. Then, Oil Red O staining images were visualized using a bright-field microscope (Nikon TE-2000U).

### Immunohistochemical analysis

After preparing the 7 groups, the animals were sacrificed using carbon dioxide and the liver tissues were extracted. The extracted tissues were fixed in 4% neutral paraformaldehyde and embedded in paraffin to prepare sections. The paraffin sections were cut to 4-µm thickness, deparaffinized, and then rehydrated. For antigen exposure, slides were autoclaved in a 10 mM sodium citrate buffer (pH 6.0). To block endogenous peroxidase activity prior to peroxidase IHC analysis, the tissues were incubated with 0.3% H₂O₂ for 10 minutes. The incubated sections were treated with phosphate-buffered saline (PBS) containing 10% NGS and 0.3% Tween-20 for 1 hour at room temperature, treated with primary antibodies of anti-PPARγ (1:50; Abcam), and incubated overnight at 4 °C.

The sections treated with the primary antibodies were washed with PBS and incubated with biotinylated anti-mouse (1:400; Dako) or anti-rabbit (1:400; Dako) secondary antibodies for 1 hour at room temperature. The sections incubated with the secondary antibodies were stained with 3,3'-diaminobenzidine (DAB; Dako) for 3-7 minutes and then counterstained with Mayer's haematoxylin. All incubations were performed in a humid chamber. After the staining, images were taken and analyzed using a bright-field microscope (Nikon TE-2000U).

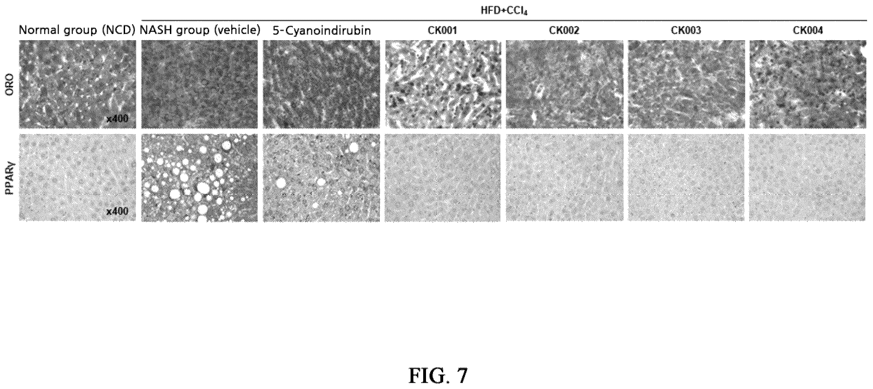

FIG. 7 shows a result of the Oil Red O staining (ORO) and immunohistochemical staining (PPARγ) showing the expression of adipocyte differentiation markers in the liver tissues extracted from the normal group (NCD), the MASH group (vehicle), and the test groups 1 to 5.

From 'ORO' in FIG. 7, lipid droplet formation and lipid infiltration can be observed. In the MASH group (vehicle), lipid droplet formation and lipid infiltration were clearly observed as compared to the normal group (NCD). In the test group 1, to which the intermediate compound (5-cyanoindirubin) was administered orally, the size and number of lipid droplets were somewhat reduced as compared to the MASH group (vehicle), but no significant difference was observed.

In the test groups 2 and 3, to which the indoline derivatives of Examples 1 and 2 were administered orally, some lipid droplet formation was observed, but it was confirmed that the size and number of lipid droplets were significantly reduced as compared to the MASH group (vehicle) and the test group 1.

In the test groups 4 and 5, to which the indoline derivatives of Examples 3 and 4 were administered orally, a significant effect of inhibiting lipid infiltration at a level similar to that of the normal group (NCD) was confirmed.

From 'PPARγ' in FIG. 7, it can be seen that the MASH group (vehicle) and the test group 1 showed significant increase in PPARγ expression as compared to the normal group (NCD). On the other hand, the test groups 4 and 5 showed a significant decrease in PPARγ expression, which was increased due to nonalcoholic fatty liver disease induced by high-fat diet and CCl₄, to the level of the normal group (NCD). PPARγ is an important transcription factor that regulates adipogenesis by binding to the aP2 promoter site during adipogenesis.

Therefore, it can be seen that the indoline derivative according to the present disclosure has CXXC5-Dvl interaction inhibition activity, thereby achieving the effect of significantly inhibiting the accumulation of lipid droplets and triglycerides induced by the high-fat diet and CCl₄.

### 3-6. Confirmation of anti-inflammatory and anti-fibrotic effects in animal model of metabolic dysfunction-associated steatohepatitis (MASH; nonalcoholic fatty liver disease/steatohepatitis)

### Immunohistochemical analysis

After preparing the 7 groups, the animals were euthanized using carbon dioxide and the liver tissues were extracted. The extracted tissues were fixed in 4% neutral paraformaldehyde and embedded in paraffin to prepare sections. The paraffin sections were cut to 4-µm thickness, deparaffinized, and then rehydrated. For antigen exposure, slides were autoclaved in a 10 mM sodium citrate buffer (pH 6.0). To block endogenous peroxidase activity prior to peroxidase IHC analysis, the tissues were incubated with 0.3% H₂O₂ for 10 minutes. The incubated sections were treated with phosphate-buffered saline (PBS) containing 10% NGS and 0.3% Tween-20 for 1 hour at room temperature, treated with the following primary antibodies, and incubated overnight at 4 °C.

Anti-PPARγ (1:50; Abcam), anti-F4/80 (1:50; Santa Cruz), anti-CD11b (1:100; Abcam), anti-α-SMA (1:100; Abcam), anti-collagen I (1:100; Abcam), anti-desmin (1:100; Abcam), anti-β-catenin (1:200; Abcam), anti-CXXC5 (1:100; Abcam).

The sections treated with the primary antibodies were washed with PBS and incubated with biotinylated anti-mouse (1:400; Dako) or anti-rabbit (1:400; Dako) secondary antibodies for 1 hour at room temperature. The sections incubated with the secondary antibodies were stained with 3,3'-diaminobenzidine (DAB; Dako) for 3-7 minutes and then counterstained with Mayer's hematoxylin. All incubations were performed in a humid chamber. After the staining, images were taken and analyzed using a bright-field microscope (Nikon TE-2000U).

### Collagen staining (Picrosirius red)

After preparing the 7 groups, the animals were euthanized using carbon dioxide and the liver tissues were extracted. The extracted tissues were fixed in 4% neutral paraformaldehyde and embedded in paraffin to prepare sections. The paraffin sections were cut to 4-µm thickness, deparaffinized, and then rehydrated. The hydrated sections were stained with a Weigert's iron hematoxylin solution for 8 minutes and then with Picrosirius red for 1 hour. Collagen fibers were stained red and nuclei were stained blue. The sections were photographed using a bright-field microscope (Nikon TE-2000U).

Through these experiments, the anti-inflammatory effect of the indoline derivatives of Examples 1 to 4 were analyzed in an animal model of nonalcoholic fatty liver disease. One of the main etiologies of nonalcoholic fatty liver disease (MASH) is the increase in lipids in liver tissues and the activation of Toll-like receptors on Kupffer stellate cells, which are macrophages present in the liver. Activated Kupffer stellate cells secrete pro-inflammatory cytokines within the lobules, causing an inflammatory response. To this end, immunohistochemical staining of F4/80 and CD11b, which are markers of Kupffer stellate cells, was performed to confirm the degree of activation of Kupffer stellate cells in order to compare the degree of inflammatory response in liver tissues and the degree of MASH progression.

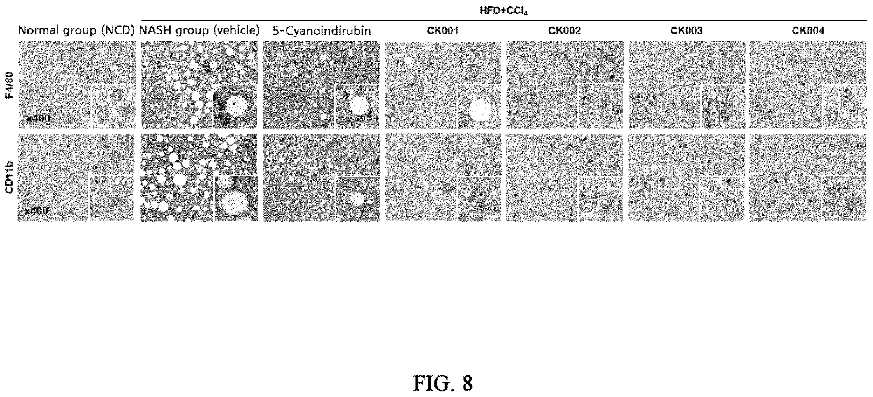

FIG. 8 shows the result of immunohistochemical staining confirming the increased expression of inflammatory markers (CD11b and F4/80) in the liver tissues extracted from the normal group (NCD), the MASH group (vehicle), and the test groups 1 to 5.

As shown in FIG. 8, it can be confirmed that the liver tissue of the MASH group (vehicle) showed a significantly increased inflammatory response due to the activation of Kupffer stellate cells as compared to the liver tissue of the normal group (NCD). In the test group 1, to which 5-cyanoindirubin, the intermediate compound of Example 1-1, was administered orally, no significant difference was observed from the MASH group (vehicle). On the other hand, it was confirmed that the liver tissues of test groups 2 to 5, to which the indoline derivatives of Examples 1 to 4 were administered orally, showed a significant decrease in the expression of F4/80 and CD11b as compared to the liver tissue of the MASH group, indicating that inflammatory response was alleviated significantly.

Therefore, it can be seen that the indoline derivatives of Examples 1 to 4 according to the present disclosure effectively suppress the inflammatory response caused by metabolic diseases through CXXC5-Dvl interaction inhibition activity.

Stellate cells exist in either a resting or activated state. Hepatic stellate cells in a resting state function to store vitamin A in lipid droplets. But, when the liver is damaged, the hepatic stellate cells become activated and participate in the accumulation and formation of collagen, causing liver fibrosis and cirrhosis. To evaluate the effect of the indoline derivative according to the present disclosure on hepatic fibrosis and stellate cells, the final stage of MASH, the expression of α-smooth muscle actin (α-SMA), collagen type I, and collagen type III (desmin), which are induced when hepatic stellate cells differentiate into myofibroblasts, was confirmed through immunohistochemical staining.

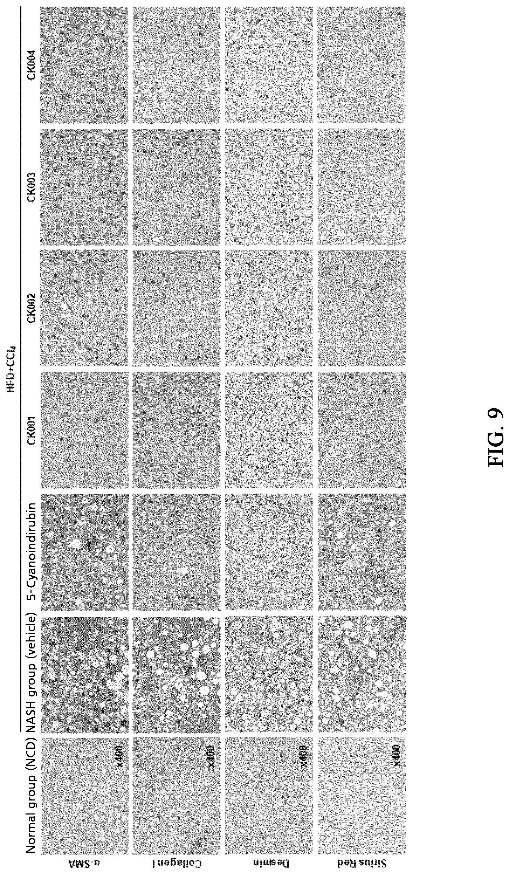

FIG. 9 shows the result of immunohistochemical staining confirming the increased expression of the fibrosis markers (α-SMA, collagen I, and desmin) in the liver tissues extracted from the normal group (NCD), the MASH group (vehicle), and the test groups 1 to 5.

As shown in FIG. 9, the MASH group (vehicle) showed significant increase in collagen production and α-SMA expression as compared to the normal group (NCD). The test group 1, to which 5-cyanoindirubin, the intermediate compound of Example 1-1, was administered orally, did not show any significant difference from the MASH group (vehicle).

On the other hand, the test groups 2 to 5, to which the indoline derivatives of Examples 1 to 4 were administered orally, showed significant decrease in collagen production and α-SMA expression as compared to the MASH group (vehicle), and it was confirmed that fibrosis was alleviated to a level similar to that of the normal group (NCD).

### 3-7. Confirmation of anti-fibrotic effect in metabolic dysfunction-associated steatohepatitis (MASH; nonalcoholic fatty liver disease/steatohepatitis)

The main mechanism of nonalcoholic fatty liver disease is the induction of hepatic stellate cell activation, which induces collagen production and leads to liver fibrosis due to collagen accumulation. From the result of collagen staining (Picrosirius red) for observing the total amount of collagen deposition in Test Example 3-6, the liver fibrosis score was analyzed. The fibrosis score was classified into four criteria as shown in Table 2. Stages 2 to 3 were diagnosed as nonalcoholic fatty liver disease (MASH) (M. Brunt et al., 2005).

**[Table 2]**

| | |
|---|---|
| Stage 1 | Locally distributed zone 3 perisinusoidal/pericellular fibrosis |
| Stage 2 | Centrally or peripherally formed zone 3 perisinusoidal/pericellular fibrosis and portal fibrosis. |
| Stage 3 | Zone 3 Perisinusoidal/pericellular fibrosis and portal fibrosis forming bridging centrally or peripherally. |
| Stage 4 | Cirrhosis |

All quantitative data are presented as mean ± standard deviation (SD). In each experiment, all measurements were made at least three times, and data were analyzed using the two-tailed, unpaired Student's t test. P values of < 0.05 were considered statistically significant.

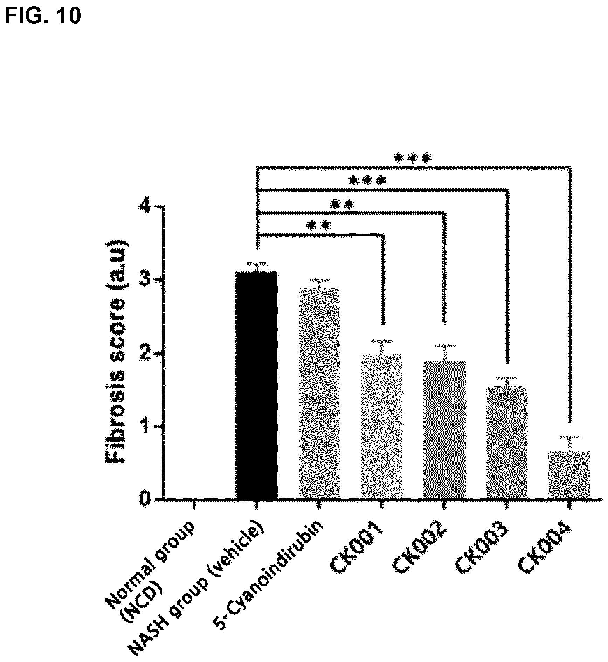

FIG. 10 shows the result of analyzing the degree of liver fibrosis for the normal group (NCD), the MASH group (vehicle), and the test groups 1 to 5. The numerical values are summarized in Table 3 below.

**[Table 3]**

| Category | Normal group (NCD) | MASH group | Test group 1 5-cyanoin dirubin | Test group 2 CK001 | Test group 3 CK002 | Test group 4 CK003 | Test group 5 CK004 |
|---|---|---|---|---|---|---|---|
| Degree of liver fibrosis (fibrosis score) | 0 | 3.11 ± 0.11 | 2.89 ± 0.11 | 2.00 ± 0.19 | 1.85 ± 0.22 | 1.56 ± 0.11 | 0.67 ± 0.19 |

As shown in FIG. 10, the MASH group (vehicle) and the test group 1 showed liver fibrosis scores corresponding to stage 3, indicating that nonalcoholic fatty liver disease progressed significantly. On the other hand, the test groups 2 to 5, to which the indoline derivatives of Examples 1 to 4 were administered orally, showed significantly and markedly alleviated liver fibrosis levels, corresponding to stage 2 to 1 or lower. In particular, the test group 5, to which the indoline derivative of Example 4 was administered orally, showed a liver fibrosis score of 0.67, confirming that liver health had recovered to the level of the normal group (NCD).

### 3-8. Confirmation of target specificity for CXXC5 in metabolic dysfunction-associated steatohepatitis (MASH; nonalcoholic fatty liver disease/steatohepatitis)

The indoline derivatives of Examples 1 to 4, which have excellent effects as CXXC5-Dvl inhibitors, were confirmed to have excellent effect of alleviating and treating metabolic diseases through the experiments described above. It was further investigated whether the therapeutic effect is associated with the activation of β-catenin expression through restoration of the Wnt/β-catenin signaling pathway.

After preparing the 7 groups, the animals were euthanized using carbon dioxide and the liver tissues were extracted. The extracted tissues were fixed in 4% neutral paraformaldehyde and embedded in paraffin to prepare sections. The paraffin sections were cut to 4-µm thickness, deparaffinized, and then rehydrated. For antigen exposure, slides were autoclaved in a 10 mM sodium citrate buffer (pH 6.0). To block endogenous peroxidase activity prior to peroxidase IHC analysis, the tissues were incubated with 0.3% H₂O₂ for 10 minutes. The incubated sections were treated with phosphate-buffered saline (PBS) containing 10% NGS and 0.3% Tween-20 for 1 hour at room temperature, treated with primary antibodies of anti-β-catenin (1:200; Abcam) and anti-CXXC5 (1:100; Abcam), and then incubated overnight at 4 °C.

The sections treated with the primary antibodies were washed with PBS and incubated with biotinylated anti-mouse (1:400; Dako) or anti-rabbit (1:400; Dako) secondary antibodies for 1 hour at room temperature. The sections incubated with the secondary antibodies were stained with 3,3'-diaminobenzidine (DAB; Dako) for 3-7 minutes and then counterstained with Mayer's hematoxylin. All incubations were performed in a humid chamber. After the staining, images were taken and analyzed using a bright-field microscope (Nikon TE-2000U).

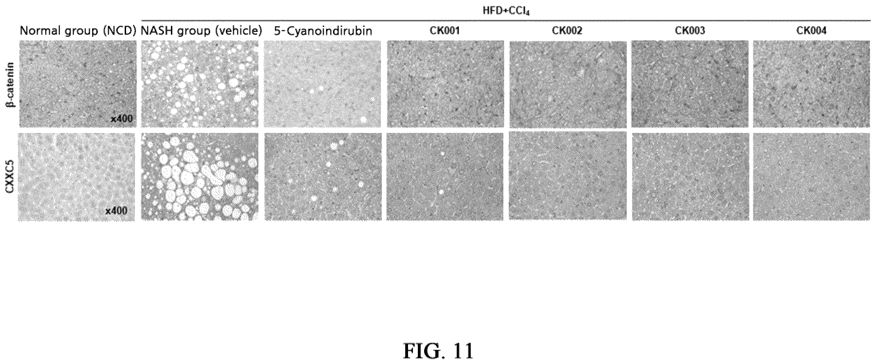

FIG. 11 shows the result of immunohistochemical staining analysis of the expression level of β-catenin and CXXC5 in the normal group (NCD), the MASH group (vehicle), and the test groups 1 to 5.

As shown in FIG. 11, the normal group (NCD) showed low CXXC5 expression and activated β-catenin expression. On the other hand, the liver tissue of the MASH group (vehicle) showed overexpression of the CXXC5 protein as compared to the liver tissue of the normal group (NCD), and the β-catenin expression was reduced because of binding of CXXC5 to the Dvl protein.

It was confirmed that CXXC5 expression was increased in the liver tissues of the test groups 2 to 5, to which the indoline derivatives of Examples 1 to 4 were administered orally, similarly to the MASH group. However, it can be seen that the expression of β-catenin was restored to a normal level.

Through this, it can be confirmed that the indoline derivatives according to the present disclosure (Examples 1 to 4) do not directly regulate CXXC5 expression itself, but rather play a role in interfering with the interaction with the Dvl protein increased due to the expression of CXXC5. Specifically, when the indoline derivative of the present disclosure is administered in a state where nonalcoholic steatohepatitis (MASH) is induced by a high-fat diet and CCl₄, and thus CXXC5 is overexpressed, the binding between the overexpressed CXXC5 and the Dvl protein is inhibited and prevented. Therefore, the Wnt/β-catenin signaling pathway is restored gradually, and the expression of β-catenin is restored to a normal level, thereby alleviating or beneficially altering the symptoms of the disease.

Therefore, the indoline derivatives of the present disclosure, particularly the indoline derivatives of Examples 1 to 4, have the effect of preventing the disease from being induced by preventing the binding and interaction with the Dvl protein when exposed to a pathological situation that causes the overexpression of MASH and CXXC5. Even when the disease is induced, symptoms may be alleviated and reduced by inhibiting further binding of CXXC5-Dvl, and the essential cause of the disease may be removed ultimately.

In contrast, the test group 1, in which the intermediate compound (5-cyanoindirubin) of Example 1-1 was administered orally, showed no significant difference from the MASH group (vehicle).

### 3-9. Immunohistochemical analysis of adipose tissue in metabolic dysfunction-associated steatohepatitis (MASH; nonalcoholic fatty liver disease/steatohepatitis)

After preparing the 7 groups, the animals were euthanized and the epididymal white adipose tissue (eWAT) and subcutaneous adipose tissue (sWAT) were extracted. The extracted tissues were fixed in 4% neutral paraformaldehyde and embedded in paraffin to prepare sections. The paraffin sections were cut to 4 µm thickness and H&E staining was performed. Twenty randomly selected microscopic fields were photographed and analyzed using a Nikon bright-field optical microscope (Nikon TE-2000U).

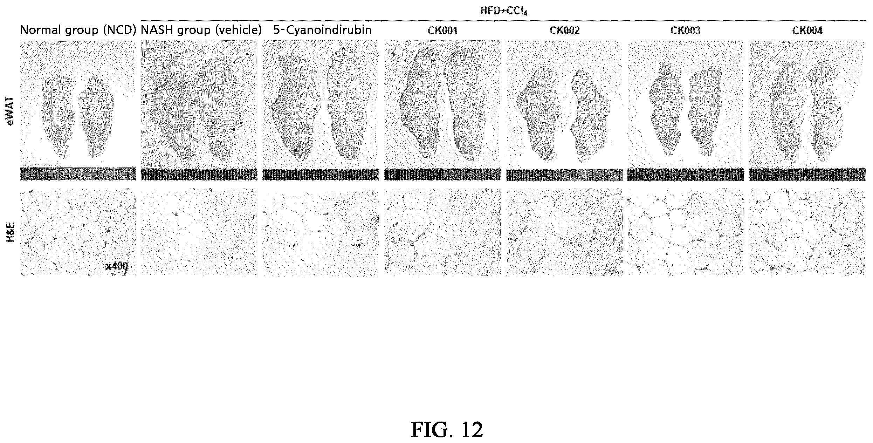

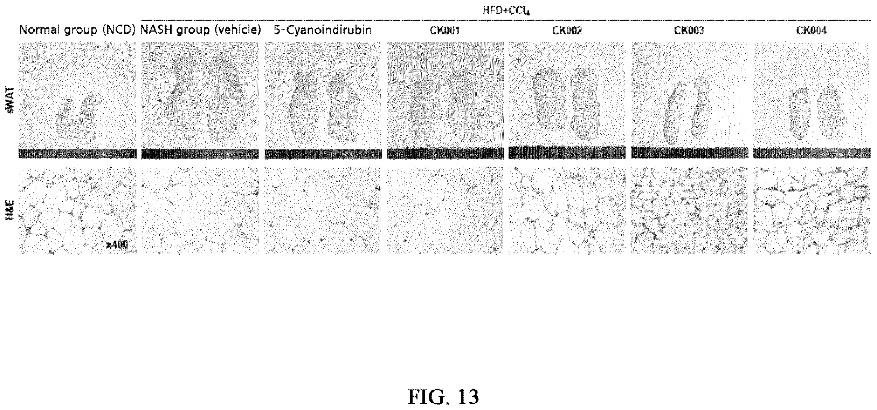

FIGS. 12 and 13 show the H&E staining images of the adipose tissues isolated from the normal group (NCD), the MASH group (vehicle), and the test groups 1 to 5. FIG. 12 shows eWAT (epididymal white adipose tissue), and FIG. 13 shows iWAT (inguinal white adipose tissue).

As shown in FIGS. 12 and 13, the size of the adipose tissue in the MASH group (vehicle) was confirmed to be significantly increased as compared to the normal group (NCD). On the other hand, the test groups 1 to 5, to which the indoline derivatives of Examples 1 to 4 were administered orally, showed significant decrease in the adipose tissue size as compared to the MASH group (vehicle). That is, it can be seen that the indoline derivative according to the present disclosure effectively suppresses adipose accumulation due to nonalcoholic fatty liver disease.

In summary of the above experimental results, it was confirmed that the novel indoline derivative according to the present disclosure, although it is a low-molecular-weight compound, can specifically and directly inhibit and suppress the binding of the CXXC5 protein and the Dvl protein, and thereby treat, prevent or alleviate related diseases. It was also confirmed that various related symptoms can be treated and alleviated significantly.

The foregoing description of the present disclosure is for illustrative purposes only, and those skilled in the art will appreciate that the present disclosure can be easily modified into other specific forms without changing the technical idea or essential characteristics of the present disclosure. Therefore, it should be understood that the test examples described above are exemplary, not limitative, in all respects and.

### Preparation Example 1. Preparation of powder

10 mg of indoline derivative of Chemical Formula 1 (any one of Examples 1 to 15)
100 mg of sucrose
10 mg of talc

The above ingredients were powdered, mixed, and then filled into a sealed pouch to prepare a powder.

### Preparation Example 2. Preparation of tablet

10 mg of indoline derivative of Chemical Formula 1 (any one of Examples 1 to 15)
100 mg of starch
100 mg of sucrose
2 mg of magnesium stearate

After mixing the above ingredients, a tablet was prepared by compressing the mixture according to a common method for preparing a tablet.

### Preparation Example 3. Preparation of capsule

10 mg of indoline derivative of Chemical Formula 1 (any one of Examples 1 to 15)
3 mg of crystalline cellulose
15 mg of lactose
1 mg of magnesium stearate

The above ingredients were mixed according to a common method for preparing a capsule, and then filled into a gelatin capsule to prepare a capsule.

### Preparation Example 4. Preparation of granule

10 mg of indoline derivative of Chemical Formula 1 (any one of Examples 1 to 15)
50 mg of soybean extract
200 mg of glucose
500 mg of starch

After mixing the above ingredients and adding 100 mL of 30% ethanol, the mixture was dried at 60° C to form granules, which were then filled in a pouch.

### Preparation Example 5. Preparation of pill

20 mg of indoline derivative of Chemical Formula 1 (any one of Examples 1 to 15)
1,500 mg of lactose
1,500 mg of glycerin
980 mg of starch

After mixing the above ingredients, the mixture was prepared into pills using a common method for preparing pills, such that each pill weighs 4 g.

### Preparation Example 6. Preparation of injection

10 mg of indoline derivative of Chemical Formula 1 (any one of Examples 1 to 15)
180 mg of mannitol
2,970 mg of sterile distilled water for injection
30 mg of Na₂HPO₄·12H₂O

The above ingredients were mixed and transferred into an ampoule (2 mL) according to a common method for preparing an injection.

### Preparation Example 7. Preparation of liquid

10 mg of indoline derivative of Chemical Formula 1 (any one of Examples 1 to 15)
10,000 mg of high-fructose corn syrup
5,000 mg of mannitol
Adequate amount of purified water

The above ingredients were dissolved in purified water according to a common liquid preparation method. Then, after adding an appropriate fragrance, the mixture was filled into a bottle and then sterilized.

## Claims

1. An indoline derivative represented by Chemical Formula 1, a hydrate thereof, a solvate thereof, or a pharmaceutically acceptable salt thereof: 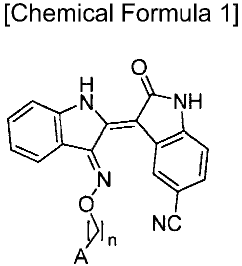 wherein
A is a 5- to 7-membered aromatic or non-aromatic heterocyclic group comprising 1 to 3 heteroatoms selected from O, N, S, SO and SO₂;
the aromatic or non-aromatic heterocyclic group may be substituted or unsubstituted with one or more substituents selected from the group consisting of hydrogen, a halogen group, a hydroxyl group (-OH), a carboxyl group, and a C₁₋₆ alkyl group; and
n is an integer from 1 to 4.

2. The indoline derivative, the hydrate thereof, the solvate thereof, or the pharmaceutically acceptable salt thereof according to claim 1, wherein,
in Chemical Formula 1, A is a 5- to 6-membered non-aromatic heterocyclic group comprising 1 to 2 heteroatoms selected from O, N, S, SO and SO₂, and
the aromatic or non-aromatic heterocyclic group is substituted or unsubstituted with one or more substituents selected from hydrogen, a carboxyl group and a C₁₋₆ alkyl group.

3. The indoline derivative, the hydrate thereof, the solvate thereof, or the pharmaceutically acceptable salt thereof according to claim 2, wherein, in Chemical Formula 1, A is selected from substituted or unsubstituted pyrrolidine, substituted or unsubstituted piperidine, substituted or unsubstituted piperazine, substituted or unsubstituted morpholine, substituted or unsubstituted thiomorpholine, and substituted or unsubstituted proline.

4. The indoline derivative, the hydrate thereof, the solvate thereof, or the pharmaceutically acceptable salt thereof according to claim 1, wherein the indoline derivative of Chemical Formula 1 is any one selected from the group consisting of
5-cyanoindirubin-3'-piperazinylethyloxime;
5-cyanoindirubin-3'-morpholinylethyloxime;
5-cyanoindirubin-3'-thiomorpholinylethyloxime;
5-cyanoindirubin-3'-(4-methylpiperazinyl)ethyloxime;
5-cyanoindirubin-3'-piperidinylethyloxime;
5-cyanoindirubin-3'-pyrrolidinylethyloxime;
5-cyanoindirubin-3'-piperidinylpropyloxime;
5-cyanoindirubin-3'-pyrrolidinylpropyloxime;
5-cyanoindirubin-3'-piperazinylpropyloxime;
5-cyanoindirubin-3'-morpholinylpropyloxime;
5-cyanoindirubin-3'-(4-methylpiperazinyl)propyloxime;
5-cyanoindirubin-3'-thiomorpholinylpropyloxime;
5-cyanoindirubin-3'-(1,1-dioxothiomorpholinyl)ethyloxime;
5-cyanoindirubin-3'-(1-oxothiomorpholinyl)ethyloxime hydrochloride; and
5-cyanoindirubin-3'-(2-prolinyl)ethyloxime.

5. The indoline derivative, the hydrate thereof, the solvate thereof, or the pharmaceutically acceptable salt thereof according to claim 1, wherein the indoline derivative of Chemical Formula 1 is any one selected from the group consisting of
5-cyanoindirubin-3'-piperazinylethyloxime;
5-cyanoindirubin-3'-morpholinylethyloxime;
5-cyanoindirubin-3'-thiomorpholinylethyloxime; and
5-cyanoindirubin-3'-(4-methylpiperazinyl)ethyloxime.

6. A method for preparing an indoline derivative represented by Chemical Formula 1, comprising:
1) a step of replacing the hydrogen atom of a hydroxyl group of an indirubin oxime derivative represented by Chemical Formula 2 with a C₁₋₆ alkyl group substituted with a C₁₋₆ haloalkyl group, a tosyl group (OTs) or a cyano group; and
2) a step of adding a 5- to 7-membered aromatic or non-aromatic heterocyclic compound comprising 1 to 3 heteroatoms selected from O, N, S, SO and SO₂ to the resultant product of the step 1) to obtain an indoline derivative represented by Chemical Formula 1: 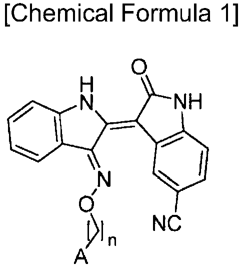 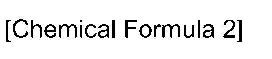 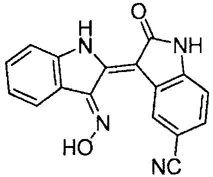 wherein
A is a 5- to 7-membered aromatic or non-aromatic heterocyclic group comprising 1 to 3 heteroatoms selected from O, N, S, SO and SO₂;
the aromatic or non-aromatic heterocyclic group may be substituted or unsubstituted with one or more substituents selected from the group consisting of hydrogen, a halogen group, a hydroxyl group (-OH), a carboxyl group, and a C₁₋₆ alkyl group; and
n is an integer from 1 to 4.

7. The preparation method according to claim 6, wherein the indirubin oxime derivative represented by Chemical Formula 2 is synthesized through the following steps:
A) a step of reacting 3-indoxyl acetate and an isatin compound represented by Chemical Formula 3 in a solvent to obtain an intermediate compound represented by Chemical Formula 4; and
B) a step of mixing the intermediate compound represented by Chemical Formula 4 with hydroxylamine to obtain an indirubin oxime derivative represented by Chemical Formula 2:

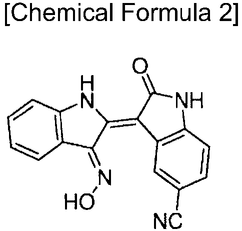

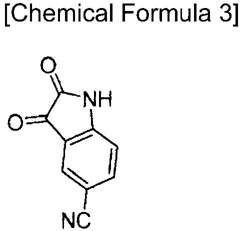

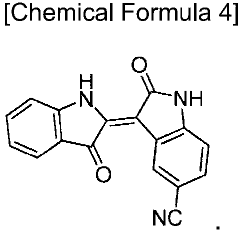

8. An intermediate compound represented by Chemical Formula 4 for preparing the indoline derivative of Chemical Formula 1 according to claim 1:

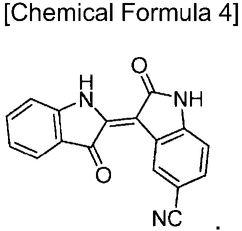

9. A pharmaceutical composition for preventing or treating a disease associated with CXXC5-Dvl interaction, comprising the indoline derivative, the hydrate thereof, the solvate thereof, or the pharmaceutically acceptable salt thereof according to claim 1 as an active ingredient.

10. The pharmaceutical composition according to claim 9, wherein the disease associated with CXXC5-Dvl interaction is at least one selected from the group consisting of metabolic diseases, bone diseases, neurodegenerative diseases, hair loss, and wound.

11. The pharmaceutical composition according to claim 10, wherein
the metabolic disease is at least one selected from the group consisting of diabetes, diabetic complications, obesity, hyperlipidemia, fatty liver, arteriosclerosis, nephropathy, hepatopathy, hypertension, stroke, heart failure and renal failure, dyslipidemia, and insulin resistance syndrome,
the bone disease is at least one selected from the group consisting of osteoporosis, osteoarthritis, osteogenesis imperfecta, bone defects, osteoclastosis, osteomalacia, osteoarthritis, rheumatoid arthritis, and fracture,
the neurodegenerative disease is at least one selected from the group consisting of Parkinson's disease, stroke, spinal cord injury, ischemic brain disease, epilepsy, Alzheimer's disease, dementia, depression, bipolar disorder, and schizophrenia,
the hair loss is at least one selected from the group consisting of androgenetic alopecia, alopecia areata, alopecia totalis, traction alopecia, cicatricial alopecia, telogen effluvium, age-related alopecia, and anagen effluvium, and
the wound is at least one selected from the group consisting of the burn of the epidermis, dermis or subcutaneous tissue of the skin, an ulcer, a trauma, a surgical operation, a plastic surgery, an implant, a childbirth, a chronic wound, a damage caused by dermatitis, a burn ulcer, a bedsore, and a chronic dermatitis.

12. The pharmaceutical composition according to claim 11, wherein
the diabetic complication is at least one selected from the group consisting of diabetic nephropathy, diabetic hepatopathy, diabetic wound, diabetic foot ulcers, hypertension, arteriosclerosis, stroke, and heart failure, and
the fatty liver is at least one selected from the group consisting of nonalcoholic fatty liver disease, nonalcoholic steatohepatitis, metabolic steatohepatitis, nonalcoholic fatty liver-associated hepatocirrhosis, and cirrhosis.

13. A food composition for preventing or alleviating a disease associated with CXXC5-Dvl interaction, comprising the indoline derivative, the hydrate thereof, the solvate thereof, or the pharmaceutically acceptable salt thereof according to claim 1 as an active ingredient.

14. A cosmetic composition for preventing or alleviating a disease associated with CXXC5-Dvl interaction, comprising the indoline derivative, the hydrate thereof, the solvate thereof, or the pharmaceutically acceptable salt thereof according to claim 1 as an active ingredient.
